# EUROPÄISCHE PATENTANMELDUNG

(11) **EP 2 051 078 A1**
(43) Veröffentlichungstag der Anmeldung: **22.04.2009**
(21) Anmeldenummer: 07118843.7
(22) Anmeldetag: 19.10.2007
(51) Int. Cl.: G01N 33/68

(54) **Verfahren und Marker zur Diagnose von Diabetes Mellitus**

(71) Anmelder: mosaiques diagnostics and therapeutics AG, 30625 Hannover (DE)
(72) Erfinder: Mischak, Harald., Prof. Dr., 31319 Sehnde (DE)
(74) Vertreter: von Kreisler Selting Werner

(57) **Zusammenfassung**

Verfahren zur Diagnostik von Diabetes mellitus umfassend den Schritt der Bestimmung einer An- oder Abwesenheit oder Amplitude von mindestens drei Polypeptidmarkern in einer Urinprobe, wobei die Polypeptidmarker ausgewählt sind aus den Markern, die in Tabelle 1 durch Werte für die Molekularmassen und die Migrationszeit charakterisiert sind.

## Beschreibung

Die vorliegende Erfindung betrifft die Diagnose von Diabetes mellitus durch nicht-invasive Analyse von endogenen Proteinen und Peptiden.

Die Anzahl an Patienten mit Diabetes mellitus ist in den letzten Jahren stark angestiegen. Diese Erkrankung stellt eines der größten Problem für die Gesundheitssysteme dar.

Diabetes Mellitus ist in einer frühen Phase noch gut klinisch zu kontrollieren, daher ist eine Früherkennung sehr wichtig. Frühe Erkennung und eine Therapie, die genau an die spezielle Erkrankung angepasst ist, können das Risiko verringern, dass die Patienten weitere Folgeerkrankungen erleiden und z.B. erblinden oder dialysepflichtig werden. Darüber hinaus reduziert eine zielgerichtete Therapie auch das hohe cardiovaskuläre Erkrankungsrisiko solcher Patienten.

Zur Zeit beruht die Diagnose auf Messungen des Blutzuckerspiegels, sowie auf dem oralen Glucose Toleranztest (OGTT).

Urinanalysen sind ein weiterer Ansatz zur Diagnose von Diabetes Mellitus. Allerdings wird heutzutage nur Glukose im Urin gemessen. Der diagnostische Wert dieser Analysen ist begrenzt, da es an einer hinreichenden Sensitivität und Selektivität fehlt. Ein möglicher Ansatz ist die Untersuchung von Proteinen und Peptiden im Urin.

Verschieden Versuche sind unternommen worden, um Proteine im Urin zu charakterisieren.

V. Thongboonkerd et al., Kidney International, Vol. 63 (2001) Seiten 1461-1469 beschreiben zweidimensionale Polyacrylamidgelelektrophorese (2D-PAGE) in Verbindung mit matrix-assisted laser desorption ionization-time-offlight mass spectrometry (MALDI-TOF) gefolgt von Massenfingerprinting, um Urinproben zu untersuchen. Insgesamt wurden 67 Formen von 47 einzelnen Proteinen identifiziert.

C.S. Spahr et al., Proteomics Vol. 1, (2001) Seiten 93-107 haben Proteine aus Urinproben mit Trypsin gespalten und konnten 751 Peptide aus 124 Proteinen mittels Flüssigchromatographie und Tandemmassenspektrometrie ermitteln.

Diese Untersuchung betraf nur gesunde Probanden. Die Untersuchungen haben sich nicht damit befasst, ob Änderungen in der Zusammensetzung von Polypeptiden im Urin zur Diagnose von Diabetes mellitus geeignet sind.

H. Mischak et al. in Clinical Science 107 (2004) Seiten 485-495 beschreiben die Proteomanalyse zur Bewertung von diabetischen Nierenschäden. Es wurden eine geringe Anzahl an Peptiden identifiziert, die mit dem Fortschreiten der Erkrankung häufiger bzw. mit geringerer Häufigkeit vorkommen.

Aufgrund der geringen Anzahl von Proben kann in diesen Arbeiten keine hinreichende Spezifität für die Diagnose erreicht werden. Daher ist weiterhin Bedarf an einer schnellen und einfachen Methode zur Diagnose von Diabetes mellitus.

Es ist daher die Aufgabe der vorliegenden Erfindung, Verfahren und Mittel für die Diagnose von Diabetes mellitus bereit zu stellen.

Gelöst wird die Aufgabe durch ein Verfahren zur Diagnostik von Diabetes mellitus umfassend den Schritt der Bestimmung einer An- oder Abwesenheit oder Amplitude von mindestens drei Polypeptidmarkern in einer Urinprobe, wobei die Polypeptidmarker ausgewählt sind aus den Markern, die in Tabelle 1 durch Werte für die Molekularmassen und die Migrationszeit charakterisiert sind.

Durch die erfindungsgemäßen Marker ist es möglich, für Diabetes mellitus, eine Spezifität und Sensitivität von mindestens 60, bevorzugt mindestens 70, mehr bevorzugt 80, noch mehr bevorzugt mindestens 90 und am meisten bevorzugt mindestens 95% zu erreichen.

Die Auswertung der gemessenen Polypeptide kann anhand der An- oder Abwesenheit oder Amplitude der Marker unter Berücksichtigung der folgenden Grenzwerte erfolgen:

"Kontrolle" bedeutet Gesunde Probanden und Patienten mit verschiedenen Erkrankungen, welche nicht an Diabetes mellitus erkrankt sind.

In den Tabellen bedeutet "MEAN" den arithmetrischen Durchschnitt, d.h. die Summe aller Werte geteilt durch die Anzahl an Werte.

"MEDIAN" ist der mittlere Wert einer Liste, d.h. der kleinste Wert, bei dem mindestens die Hälfte der Werte in der Liste nicht größer sind. Bei einer ungeraden Anzahl an Werte ist der Median der mittlere Wert einer nach aufsteigender Reihenfolge sortierten Liste. Bei einer geraden Anzahl an Werten ist der Median die Summe der beiden nach einer Sortierung erhaltenen mittleren Werte geteilt durch 2.

AUC ist das sogenannte "Area-under-the-curve", welches im Rahmen der ROC (receiver-operator-characteristic) Analyse untersucht wird, und welches ein Mass für die Qualität des einzelnen Parameters (Biomarkers), bezogen auf die untersuchten Fälle, angibt. Hierbei wird im Diagramm die Sensitivität auf der Ordinate gegen 1-Spezifität auf der Abszisse aufgetragen. Spezifität ist definiert als die Nummer der tatsächlich negativen Proben geteilt durch die Summe der Anzahl der tatsächlich Negativen und der Anzahl der Falsch-Positiven. Eine Spezifität von 1 bedeutet, dass ein Test alle gesunden Personen als gesund erkennt, d.h. kein Gesunder wird als krank identifiziert. Dies trifft keine Aussage darüber, wie gut der Test kranke Patienten erkennt. Sensitivität ist definiert als die Anzahl der tatsächlichen positiven Proben geteilt durch die Summe der Anzahl der tatsächlich Positiven und die Anzahl der Falsch-Negativen. Eine Sensitivität von 1 bedeutet, dass der Test alle Kranken erkennt. Er trifft keine Aussage, wie gut der Test gesunde Personen erkennt. Ein AUC-Wert von 1 bedeutet mithin, dass alle Proben richtig zugeordnet wurden (Spezifität und Sensitivität von 1), ein AUC-Wert von 0,5 bedeutet, dass die Proben mit Ratewahrscheinlichkeit zugeordnet wurden, mithin der Parameter keine Aussagekraft beinhaltet.

Die Methode von "Bonferoni" wird eingesetzt, um multivariable statistische Analysen zu korrigieren und ergibt einen korrigierten p-Wert. Der p-Wert gibt die Wahrscheinlichkeit an, dass die gefundenen Unterschiede auf zufällige Schwankungen zurückgehen. Je kleiner der p-Wert, desto höher die Aussagekraft.

Bevorzugt werden Marker eingesetzt, deren AUC-Wert größer 0,6, bevorzugt größer 0,7 ist und/oder deren Bonferoni korrigierte p-Wert kleiner 10⁻⁴, bevorzugt kleiner 10⁻⁵, noch mehr bevorzugt kleiner 10⁻⁶ ist.

Die Migrationszeit wird mittels Kapillarelektrophorese (capillary electrophoresis, CE) - wie z.B. in Beispiel unter Punkt 2 ausgeführt - bestimmt. In diesem Beispiel wird eine 90 cm lange Glaskapillare mit einem inneren Durchmesser (ID) von 50 µm und einem äußeren Durchmesser (OD) von 360 µm bei einer angelegten Spannung von 30 kV betrieben. Als Laufmittel wird zum Beispiel 30% Methanol, 0,5% Ameisensäure in Wasser verwendet.

Es ist bekannt, das die CE-Migrationszeit variieren kann. Dennoch ist die Reihenfolge, mit der die Polypeptidmarker eluieren, für jedes verwendete CE System unter den angegebenen Bedingungen typischerweise gleich. Um dennoch auftretende Unterschiede in der Migrationszeit auszugleichen, kann das System unter Verwendung von Standards, für die die Migrationszeiten genau bekannt sind, normiert werden. Diese Standards können z.B. die in den Beispielen angegebenen Polypeptide sein (siehe Beispiel Punkt 3).

Die Charakterisierung der Polypeptide, die in den Tabellen gezeigt sind, wurde mittels Kapillarelektrophorese-Massenspektrometrie (CE-MS) bestimmt, einem Verfahren, das z.B. ausführlich von Neuhoff et al. (Rapid communications in mass spectrometry, 2004, Bd. 20, Seite 149-156) beschrieben wurde. Die Variation der Molekülmassen zwischen einzelnen Messungen oder zwischen verschiedenen Massenspektrometern ist bei exakter Kalibrierung relativ klein, typischerweise im Bereich von ± 0,01% oder 0,005%.

Die erfindungsgemäßen Polypeptidmarker sind Proteine oder Peptide oder Abbauprodukte von Proteinen oder Peptiden. Sie können chemisch modifiziert sein, z.B. durch posttranslationale Modifikationen wie Glykolisierung, Phosphorylierung, Alkylierung oder Disulfidverbrückung, oder durch andere Reaktionen, z.B. im Rahmen des Abbaus, verändert sein. Darüber hinaus können die Polypeptidmarker auch im Rahmen der Aufreinigung der Proben chemisch verändert, z.B. oxidiert, sein.

Ausgehend von den Parametern, die die Polypeptidmarker bestimmen (Molekularmasse und Migrationszeit), ist es möglich, durch im Stand der Technik bekannte Verfahren die Sequenz der entsprechenden Polypeptide zu identifizieren.

Die erfindungsgemäßen Polypeptide werden verwendet, um Diabetes mellitus zu diagnostizieren.

Unter Diagnose versteht man den Vorgang der Erkenntnisgewinnung durch die Zuordnung von Symptomen oder Phänomenen zu einer Krankheit oder Verletzung. Im vorliegenden Fall wird die An- oder Abwesenheit bestimmter Polypeptidmarker auch Differentialdiagnostik genutzt. Die An- oder Abwesenheit eines Polypeptidmarkers kann durch jedes im Stand der Technik bekannte Verfahren gemessen werden. Verfahren, die verwendet werden können, sind weiter unten beispielhaft aufgeführt.

Ein Polypeptidmarker ist anwesend, wenn sein Messwert mindestens so hoch ist wie der Schwellenwert. Liegt sein Messwert darunter, ist der Polypeptidmarker abwesend. Der Schwellenwert kann entweder durch die Sensitivität des Messverfahrens (Nachweisgrenze) bestimmt werden oder anhand von Erfahrungen definiert werden.

Im Zusammenhang mit der vorliegenden Erfindung wird der Schwellenwert vorzugsweise überschritten, wenn der Messwert der Probe für eine bestimmte Molekularmasse mindestens doppelt so hoch ist, wie der einer Leerprobe (z.B. nur Puffer oder Lösungsmittel).

Der oder die Polypeptidmarker wird/werden in der Weise verwendet, dass seine/ihre An- oder Abwesenheit gemessen wird, wobei die An- oder Abwesenheit indikativ für die Diabetes mellitus ist. So gibt es Polypeptidmarker, die typischerweise bei Individuen mit Diabetes mellitus vorhanden sind, jedoch bei Individuen ohne Diabetes mellitus seltener oder gar nicht auftreten. Weiterhin gibt es Polypeptidmarker, die bei Patienten mit Diabetes mellitus vorhanden sind, jedoch bei Patienten ohne Diabetes mellitus nicht oder nur seltener vorhanden sind.

Zusätzlich oder auch alternativ zu den Frequenzmarkern (Bestimmung der An- oder Abwesenheit) können auch Amplitudenmarker zur Diagnose verwendet werden. Amplitudenmarker werden in der Weise verwendet, das nicht die An oder Abwesenheit entscheidend ist, sondern die Höhe des Signals (die Amplitude) bei Anwesenheit des Signals in beiden Gruppen entscheidet. In den Tabellen sind die mittleren Amplituden der entsprechenden Signale (charakterisiert über Masse and Migrationszeit) über alle gemessenen Proben angegeben. Dabei sind zwei Nominierungsverfahren möglich, um eine Vergleichbarkeit zwischen unterschiedlich konzentrierten Proben oder unterschiedlichen Messmethoden zu erreichen. Im ersten Ansatz werden alle Peptidsignale einer Probe auf eine Gesamtamplitude von 1 Million Counts normiert. Die jeweiligen mittleren Amplituden der Einzelmarker sind daher als parts per million (ppm) angegeben.

Zusätzlich besteht die Möglichkeit über ein alternatives Normierungsverfahren weitere Amplitudenmarker zu definieren: in diesem Fall werden alle Peptidsignale einer Probe mit einem gemeinsamen Normierungsfaktor skaliert. Dazu wird eine lineare Regression zwischen den Peptid-Amplituden der einzelnen Proben und den Referenzwerten aller bekannten Polypeptide gebildet. Die Steigerung der Regressionsgeraden entspricht gerade der relativen Konzentration und wird als Normierungsfaktor für diese Probe verwandt.

Alle verwendeten Gruppen bestehen aus mindestens 100 einzelnen Patienten-oder Kontrollproben, um eine verlässliche mittlere Amplitude zu erhalten. Die Entscheidung zu einer Diagnose fällt dabei je nachdem, wie hoch die Amplitude der jeweiligen Polypeptidmarker in der Patientenprobe im Vergleich zu den mittleren Amplituden in der Kontrollgruppe bzw. der "Diabetes mellitus"-Gruppe ist. Liegt der Wert nahe an der mittleren Amplitude der "Diabetes mellitus"-Gruppe, ist von dem Vorliegen eines Diabetes mellitus auszugehen, entspricht sie eher den mittleren Amplituden der Kontroll-Gruppe, ist nicht von einem Diabetes mellitus auszugehen. Der Abstand zur mittleren Amplitude kann als eine Wahrscheinlichkeit für die Zugehörigkeit zu einer Gruppe interpretiert werden.

Alternativ kann der Abstand zwischen dem Messwert und der mittleren Amplitude als eine Wahrscheinlichkeit für die Zugehörigkeit zu einer Gruppe betrachtet werden.

Ein Frequenzmarker ist eine Variante des Amplitudenmarkers, bei dem in einigen Proben die Amplitude gering ist. Es ist möglich, solche Frequenzmarker in Amplitudenmarker umzurechnen, in dem in die Berechnung der Amplitude die entsprechenden Proben, bei denen der Marker nicht gefunden wird, mit einer sehr kleinen Amplitude - im Bereich der Nachweisgrenze - in die Berechnung eingeht.

Das Individuum, von dem die Probe stammt, in der die An- oder Abwesenheit eines oder mehrerer Polypeptidmarker bestimmt wird, kann jedes Individuum sein, das an Diabetes mellitus leiden kann. Vorzugsweise handelt es sich bei dem Individuum um ein Säugetier, am meisten bevorzugt handelt es sich um einen Menschen.

In einer bevorzugten Ausführungsform der Erfindung werden nicht nur drei Polypeptidmarker, sondern eine größere Kombination von Markern verwendet, um eine exaktere Diagnose zu ermöglichen. Durch Vergleich einer Mehrzahl von Polypeptidmarkern kann die Verfälschung des Gesamtergebnisses durch einzelne individuelle Abweichungen von der typischen Anwesenheitswahrscheinlichkeit im einzelnen Individuum reduziert oder vermieden werden.

Bei der Probe, in der die An- oder Abwesenheit des oder der erfindungsgemäßen Polypeptidmarker gemessen werden, kann es sich um jede Probe handeln, die aus dem Körper des Individuums gewonnen wird. Bei der Probe handelt es sich um eine Probe, die über eine Polypeptidzusammensetzung verfügt, die geeignet ist, Aussagen über den Zustand des Individuums zu treffen. Beispielsweise kann es sich um Blut, Urin, eine Gelenkflüssigkeit, eine Gewebeflüssigkeit, ein Körpersekret, Schweiß, Liquor, Lymphe, Darm-, Magen-, Pankreassaft, Galle, Tränenflüssigkeit, eine Gewebeprobe, Sperma, Vaginalflüssigkeit oder eine Stuhlprobe handeln. Vorzugsweise handelt es sich um eine Flüssigprobe.

In einer bevorzugten Ausführungsform handelt es sich bei der Probe um eine Urinprobe.

Urinproben können wie im Stand der Technik bekannt genommen werden. Vorzugsweise wird im Zusammenhang mit der vorliegenden Erfindung eine Mittelstrahlurinprobe verwendet. Die Urinprobe kann z.B. mittels eines Katheters oder auch mit Hilfe eines Urinierungsapparates, wie in WO 01/74275 beschrieben, entnommen werden.

Die An- oder Abwesenheit eines Polypeptidmarkers in der Probe kann durch jedes im Stand der Technik bekannte Verfahren, das zur Messung von Polypeptidmarkern geeignet ist, bestimmt werden. Dem Fachmann sind solche Verfahren bekannt. Grundsätzlich kann die An- oder Abwesenheit eines Polypeptidmarkers durch direkte Verfahren, wie z.B. Massenspektrometrie, oder indirekte Verfahren, wie z.B. mittels Liganden, bestimmt werden.

Falls erforderlich oder wünschenswert kann die Probe des Individuums, z.B. die Urinprobe, vor der Messung der An- oder Abwesenheit des oder der Polypeptidmarker durch jedes geeignete Mittel vorbehandelt und z.B. aufgereinigt oder aufgetrennt werden. Die Behandlung kann z.B. eine Aufreinigung, Trennung, Verdünnung oder Konzentrierung umfassen. Die Verfahren können beispielsweise eine Zentrifugation, Filtration, Ultrafiltration, Dialyse, eine Fällung oder chromatographische Verfahren wie Affinitätstrennung oder Trennung mittels Ionenaustauscherchromatographie, oder eine elektrophoretische Trennung sein. Besondere Beispiele hierfür sind Gelelektrophorese, zweidimensionale Polyacrylamidgelelektrophorese (2D-PAGE), Kapillarelektrophorese, Metallaffinitätschromatographie, immobilisierte Metallaffinitätschromatographie (IMAC), Affinitätschromatographie auf der Basis von Lektinen, Flüssigchromatographie, Hochleistungsflüssigchromatographie (HPLC), Normal- und Umkehrphasen-HPLC, Kationenaustauscherchromatographie und selektive Bindung an Oberflächen. Alle diese Verfahren sind dem Fachmann gut bekannt und der Fachmann wird das Verfahren in Abhängigkeit von der verwendeten Probe und dem Verfahren zur Bestimmung der An- oder Abwesenheit des oder der Polypeptidmarker auswählen können.

In einer Ausführungsform der Erfindung wird die Probe vor ihrer Messung mittels Elektrophorese aufgetrennt, mittels Ultrazentrifugation gereinigt und/oder mittels Ultrafiltration in Fraktionen, die Polypeptidmarker bestimmter molekularer Größe enthalten, aufgetrennt.

Vorzugsweise wird ein massenspektrometrisches Verfahren verwendet, um die An- oder Abwesenheit eines Polypeptidmarkers zu bestimmen, wobei diesem Verfahren eine Aufreinigung oder Auftrennung der Probe vorgeschaltet werden kann. Die massenspektrometrische Analyse besitzt gegenüber den derzeit gängigen Verfahren den Vorteil, dass die Konzentration vieler (>100) Polypeptide einer Probe mittels einer einzigen Analyse bestimmt werden kann. Jeder Typ eines Massenspektrometers kann verwendet werden. Mit der Massenspektrometrie ist es möglich, routinemäßig 10 fmol eines Polypeptidmarkers, also 0,1 ng eines 10 kDa Proteins mit einer Messgenauigkeit von ca. ±0,01% aus einem komplexen Gemisch zu vermessen. Bei Massenspektrometern ist eine Ionen-bildende Einheit mit einem geeigneten Analysegerät gekoppelt. Zum Beispiel werden meistens Elektrospray-Ionisations (ESI) Interfaces verwendet, um Ionen aus Flüssigproben zu vermessen, wohingegen die Matrix-assisted-laser-desorption/ionisation (MALDI) Technik verwendet wird, um Ionen aus mit einer Matrix kristallisierten Probe zu vermessen. Zur Analyse der entstandenen Ionen können z.B. Quadrupole, Ionenfallen oder Time-offlight (TOF) Analysatoren verwendet werden.

Bei der Elektrosprayionisation (ESI) werden die in Lösung vorliegenden Moleküle u.a. unter dem Einfluss von Hochspannung (z.B. 1-8 kV) versprüht, wobei sich geladene Tröpfchen bilden, die durch Verdampfen des Lösungsmittels kleiner werden. Schließlich kommt es durch sog. Coulomb-Explosionen zur Bildung freier Ionen, die dann analysiert und detektiert werden können.

Bei der Analyse der Ionen mittels TOF wird eine bestimmte Beschleunigungsspannung angelegt, die den Ionen eine gleich große kinetische Energie verleiht. Dann wird sehr genau die Zeit gemessen, die die jeweiligen Ionen benötigen, um eine Driftstrecke durch das Flugrohr zurückzulegen. Da bei gleicher kinetische Energie die Geschwindigkeit der Ionen von Ihrer Masse abhängt, kann diese somit bestimmt werden. TOF-Analysatoren haben eine sehr hohe Scan-Geschwindigkeit und erreichen eine sehr hohe Auflösung.

Bevorzugte Verfahren zur Bestimmung der An- oder Abwesenheit von Polypeptid-markern schließen Gasphasenionenspektrometrie, wie Laserdesorptions /Ionisations-Massenspektrometrie, MALDI-TOF-MS, SELDI-TOF-MS (Surface enhanced laser desorption ionisation), LC-MS (Liquid chromatography- mass spectrometry), 2D-PAGE-MS und Kapillarelektrophorese-Massenspektrometrie (CE-MS) ein. Alle genannten Verfahren sind dem Fachmann bekannt.

Ein besonders bevorzugtes Verfahren ist CE-MS, in welchem die Kapillarelektrophorese mit Massenspektrometrie gekoppelt wird. Dieses Verfahren ist ausführlich z.B. in der deutschen Patentanmeldung DE 10021737, bei Kaiser et al. (J. Chromatogr. A, 2003, Bd. 1013:157-171, sowie Electrophoresis, 2004, 25:2044-2055) und bei Wittke et al. (J. Chromatogr. A, 2003, 1013:173-181) beschrieben. Die CE-MS Technik erlaubt, das Vorhandensein einiger Hunderter Polypeptidmarker einer Probe gleichzeitig in kurzer Zeit, einem geringen Volumen und hoher Sensitivität zu bestimmen. Nachdem eine Probe vermessen wurde, wird ein Muster der gemessenen Polypeptidmarker hergestellt. Dieses kann mit Referenzmustern von kranken bzw. gesunden Individuen verglichen werden. In den meisten Fällen ist es ausreichend, eine begrenzte Anzahl von Polypeptidmarkern für die Diagnostik von UAS zu verwenden. Weiter bevorzugt ist ein CE-MS Verfahren, das CE online an ein ESI-TOF-MS gekoppelt, einschließt.

Für CE-MS ist die Verwendung von flüchtigen Lösungsmitteln bevorzugt, außerdem arbeitet man am besten unter im Wesentlichen salzfreien Bedingungen. Beispiele geeigneter Lösungsmittel umfassen Acetonitril, Methanol und ähnliche. Die Lösungsmittel können mit Wasser verdünnt und mit einer Säure (z.B. 0,1% bis 1% Ameisensäure) versetzt sein, um den Analyten, vorzugsweise die Polypeptide, zu protonieren.

Mit der Kapillarelektrophorese ist es möglich, Moleküle nach ihrer Ladung und Größe zu trennen. Neutrale Teilchen wandern beim Anlegen eines Stromes mit der Geschwindigkeit des elektroosmotischen Flusses, Kationen werden zur Kathode beschleunigt und Anionen verzögert. Der Vorteil von Kapillaren in der Elektrophorese besteht im günstigen Verhältnis von Oberfläche zu Volumen, was einen guten Abtransport der beim Stromfluss entstehenden Jouleschen Wärme ermöglicht. Dies wiederum erlaubt das Anlegen hoher Spannungen (üblicherweise bis 30 kV) und damit eine hohe Trennleistung und kurze Analysezeiten.

Bei der Kapillarelektrophorese werden normalerweise Quarzglaskapillaren mit Innendurchmessern von typischerweise 50 bis 75 µm eingesetzt. Die verwendeten Längen betragen 30-100 cm. Darüber hinaus bestehen die Kapillaren in der Regel aus kunststoffumhüllten Quarzglas. Die Kapillaren können sowohl unbehandelt sei, d.h. auf der Innenseite ihre hydrophilen Gruppen zeigen, als auch auf der Innenseite beschichtet sein. Eine hydrophobe Beschichtung kann verwendet werden, um die Auflösung zu verbessern. Zusätzlich zur Spannung kann auch ein Druck angelegt werden, der typischerweise im Bereich von 0-1 psi liegt. Der Druck kann dabei auch erst während der Trennung angelegt oder währenddessen verändert werden.

In einem bevorzugten Verfahren zur Messung von Polypeptidmarkern werden die Marker der Probe mittels Kapillarelektrophorese getrennt, anschließend direkt ionisiert und online in ein daran gekoppeltes Massenspektrometer zur Detektion überführt.

In dem erfindungsgemäßen Verfahren können in vorteilhafter Weise mehrere Polypeptidmarker zur Diagnostik verwendet werden.

Bevorzugt ist die Verwendung von mindestens 5, 6, 8, oder 10 Markern.

In einer Ausführungsform werden 20 bis 50 Marker verwendet.

Besonders bevorzugt werden alle Marker verwendet, die hier beschrieben sind.

Bevorzugt werden mindestens drei Marker verwendet, deren mittlerer AUC größer 0,65 ist und deren mittlerer Bonferoni korrigierter p-Wert kleiner 10⁻⁵ ist.

Bevorzugt werden die drei oder mehr Marker so gewählt, dass der Durchschnitt der Quotienten aus AUC und Bonferoni korrigierte. p-Wert größer 1.000, bevorzugt größer 10.000, noch mehr bevorzugt größer 100.000 ist.

Um die Wahrscheinlichkeit für das Vorliegen einer Erkrankung bei Verwendung mehrerer Marker zu bestimmen, können dem Fachmann bekannte statistische Verfahren verwendet werden. Beispielsweise kann das von Weissinger et al. (Kidney Int., 2004, 65:2426-2434) beschriebene Random-Forests-Verfahren unter Verwendung eines Computerprogramms wie z.B. S-Plus oder die in der selben Veröffentlichung beschriebenen support-vector-machines verwendet werden.

Tabelle 1 gibt die Masse in Dalton und die CE-Zeit in Minuten an.

Tabelle 2 gibt die Häufigkeit der entsprechenden Marker bei Normalkontrollen (Nichtdiabetikern) und Diabetespatienten an, Mittelwerte (mean und mediam) der Amplituden sowie die Bonferoni korrigierten p-Werte.

Tabelle 3 gibt für einen Teil der Marker die Proteinsequenz und die Zuordnung zu bekannten Proteinen an.

### Beispiel:

### 1. Probenvorbereitung:

Zur Detektion der Polypeptidmarker zur Diagnostik wurde Urin verwendet. Urin wurde von gesunden Spendern und Patienten, die nicht an Diabetes mellitus leiden (Vergleichsgruppe) sowie Patienten, die an Diabetes mellitus leiden, abgenommen.

Für die nachfolgende CE-MS Messung mussten die auch in Urin von Patienten in höherer Konzentration vorkommenden Proteine wie Albumin und Immunoglobuline durch Ultrafiltration abgetrennt werden. Dazu wurden 700 µl Urin entnommen und mit 700 µl Filtrationspuffer (2M Harnstoff, 10mM Ammoniak, 0,02% SDS) versetzt. Diese 1,4 ml Probenvolumen wurden ultrafiltriert (20 kDa, Sartorius, Göttingen, DE). Die UF wurde bei 3000 U/min in einer Zentrifuge durchgeführt bis 1,1 ml Ultrafiltrat erhalten wurden.

Die erhaltenen 1,1 ml Filtrat wurden dann auf eine PD 10 Säule aufgetragen (Amersham Bioscience, Uppsala, Schweden) und gegen 2,5 ml 0,01% NH₄OH entsalzt und lyophillisert. Zur CE-MS Messung wurden die Polypeptide dann mit 20 µl Wasser (HPLC-Reinheit, Merck) resuspendiert.

### 2. CE-MS Messung:

Die CE-MS Messungen wurden mit einem Kapillarelektrophoresesystem von Beckman Coulter (P/ACE MDQ System; Beckman Coulter Inc, Fullerton, USA) und einem ESI-TOF Massenspektrometer von Bruker (micro-TOF MS, Bruker Daltonik, Bremen, D) durchgeführt.

Die CE Kapillaren wurden von Beckman Coulter bezogen, sie hatten einen ID/OD von 50/360 µm und eine Länge von 90 cm. Die mobile Phase für die CE Trennung bestand aus 20% Acetonitril und 0,25% Ameisensäure in Wasser. Für den "Sheath-Flow" am MS wurde 30% Isopropanol mit 0,5% Ameisensäure verwendet, hier mit einer Flussrate von 2 µl/min. Die Kopplung von CE und MS wurde durch ein CE-ESI-MS Sprayer Kit (Agilent Technologies, Waldbronn, DE) realisiert.

Um die Probe zu injizieren, wurde 1 bis max. 6 psi Druck angelegt, die Dauer der Injektion betrug 99 Sekunden. Mit diesen Parametern wurden ca. 150 nl der Probe in die Kapillare injiziert, dieses entspricht ca. 10% des Kapillarvolumens. Um die Probe in der Kapillare aufzukonzentrieren wurde eine "Stacking"-Technik verwendet. Dabei wird vor der Probeninjektion für 7 Sek. (bei 1 psi) eine 1M NH₃ Lösung injiziert, nach der Probeninjektion für 5 Sek. eine 2M Ameisensäurelösung. Nach Anlegen der Trennspannung (30 kV) werden die Analyten zwischen diesen Lösungen automatisch aufkonzentriert.

Die folgende CE-Trennung wurde mit einer Druckmethode durchgeführt: 40 Minuten mit 0 psi, dann für 2 min 0,1 psi, für 2 min 0,2 psi, für 2 min 0,3 psi, für 2 min 0,4 psi, abschließend 32 min bei 0,5 psi. Die Gesamtdauer eines Trennlaufes betrug damit 80 Minuten.

Um auf der Seite des MS eine möglichst gute Signalintensität zu erhalten, wurde das "Nebulizer Gas" auf den niedrigsten möglichen Wert eingestellt. Die an der Spraynadel angelegte Spannung zur Erzeugung des Elektrosprays betrug 3700 - 4100 V. Die übrigen Einstellungen am Massenspektrometer wurden gemäß Anweisung des Herstellers für Peptiddetektion optimiert. Die Spektren wurden über einen Massenbereich von m/z 400 bis m/z 3000 aufgenommen und alle 3 Sek. akkumuliert.

### 3. Standards für die CE-Messung

Zur Kontrolle und Kalibrierung der CE-Messung wurden die folgenden Proteine bzw. Polypeptide eingesetzt, welche unter den gewählten Bedingungen durch die unten aufgeführten CE-Migrationszeiten charakterisiert sind:

| Protein/Polypeptid | Migrationszeit |
|---|---|
| Aprotinin, (SIGMA, Taufkirchen, DE; Kat.Nr. A1153) | 19,3 min |
| Ribonuclease, SIGMA, Taufkirchen, DE; Kat.Nr.; R4875 | 19,55min |
| Lysozym, SIGMA, Taufkirchen, DE; Kat.Nr.; L7651 | 19,28 min |
| "REV", Sequenz: REVQSKIGYGRQIIS | 20,95 min |
| "ELM", Sequenz: ELMTGELPYSHINNRDQIIFMVGR | 23,49 min |
| "KINCON", Sequenz: TGSLPYSHIGSRDQIIFMVGR | 22,62 min |
| "GIVLY" Sequenz: GIVLYELMTGELPYSHIN | 32,2 min |

Die Proteine/Polypeptide werden jeweils in einer Konzentration von 10 pmol/µl in Wasser eingesetzt. "REV", "ELM", "KINCON" und "GIVLY" stellen synthetische Peptide dar.

Die Molekularmassen der Peptide sowie die in der MS sichtbaren m/z Verhältnisse der einzelnen Ladungszustände sind in der folgenden Tabelle angegeben:

| **H (mono)** | 1,0079 | 1,0079 | 1,0079 | 1,0079 | 1,0079 | 1,0079 | 1,0079 |
|---|---|---|---|---|---|---|---|
| m/z | **Aprotinin** | **Ribo-nuclease** | **Lysozym** | **REV** | **KINCON** | **ELM** | **GIVLY** |
| | Mono Mass | Mono Mass | Mono Mass | Mono Mass | Mono Mass | Mono Mass | Mono Mass |
| 0 | 6513,09 | 13681,32 | 14303,88 | 1732,96 | 2333,19 | 2832,41 | 2048,03 |
| 1 | 6514,0979 | 13682,328 | 14304,888 | 1733,9679 | 2334,1979 | 2833,4179 | 2049,0379 |
| 2 | 3257,5529 | 6841,6679 | 7152,9479 | 867,4879 | 1167,6029 | 1417,2129 | 1025,0229 |
| 3 | 2172,0379 | 4561,4479 | 4768,9679 | 578,6612 | 778,7379 | 945,1446 | 683,6846 |
| 4 | 1629,2804 | 3421,3379 | 3576,9779 | 434,2479 | 584,3054 | 709,1104 | 513,0154 |
| 5 | 1303,6259 | 2737,2719 | 2861,7839 | 347,5999 | 467,6459 | 567,4899 | 410,6139 |
| 6 | 1086,5229 | 2281,2279 | 2384,9879 | 289,8346 | 389,8729 | 473,0762 | 342,3462 |
| 7 | 931,4494 | 1955,4822 | 2044,4193 | 248,5736 | 334,3208 | 405,6379 | 293,5836 |
| 8 | 815,1442 | 1711,1729 | 1788,9929 | 217,6279 | 292,6567 | 355,0592 | 257,0117 |
| 9 | 724,6846 | 1521,1546 | 1590,3279 | 193,559 | 260,2512 | 315,7201 | 228,5668 |
| 10 | 652,3169 | 1369,1399 | 1431,3959 | 174,3039 | 234,3269 | 284,2489 | 205,8109 |
| 11 | 593,107 | 1244,7643 | 1301,3606 | 158,5497 | 213,1161 | 258,4997 | 187,1924 |
| 12 | 543,7654 | 1141,1179 | 1192,9979 | 145,4212 | 195,4404 | 237,0421 | 171,6771 |
| 13 | 502,0148 | 1053,4171 | 1101,3063 | 134,3125 | 180,4841 | 218,8856 | 158,5486 |

Es ist dem Fachmann prinzipiell bekannt, dass bei kapillarelektrophoretischen Trennungen geringe Schwankungen der Migrationszeiten auftreten können. Unter den beschriebenen Bedingungen ändert sich jedoch die Migrationsreihenfolge nicht. Es ist für den Fachmann in Kenntnis der angegebenen Massen und CE-Zeiten problemlos möglich, eigene Messungen den erfindungsgemäßen Polypeptidmarkern zuzuordnen. Hierzu kann er beispielsweise wie folgt vorgehen: zunächst wählt er eines der in seiner Messung gefundenen Polypeptide (Peptid 1) aus und versucht, innerhalb eines Zeitfensters der angegebenen CE-Zeit (beispielsweise ± 5 min) eine oder mehrere übereinstimmende Massen zu finden. Findet er innerhalb dieses Intervalls nur eine übereinstimmende Masse, ist die Zuordnung fertiggestellt. Findet er mehrere passende Massen, muss noch eine Entscheidung über die Zuordnung gefällt werden. Hierzu wird ein weiteres Peptid (Peptid 2) aus der Messung ausgewählt und versucht, hierfür einen passenden Polypeptidmarker zu identifizieren, wobei wieder ein entsprechendes Zeitfenster berücksichtigt wird.

Lassen sich nun wiederum mit einer entsprechenden Masse mehrere Marker finden, ist die wahrscheinlichste Zuordnung die, bei der zwischen der Verschiebung für das Peptide 1 und für das Peptid 2 ein im wesentlichen linearer Zusammenhang besteht.

In Abhängigkeit von der Komplexität des Zuordnungsproblems bietet es sich für den Fachmann an, gegebenenfalls weitere Proteine aus seiner Probe für die Zuordnung zu verwenden, beispielsweise zehn Proteine. Typischerweise sind die Migrationszeiten entweder um gewisse absolute Werte verlängert oder verkürzt oder es treten Stauchungen oder Strickungen des gesamten Verlaufs auf. Comigrierende Peptide comigrieren aber auch unter solchen Bedingungen.

Zudem kann der Fachmann sich die von Zuerbig et al. in Electrophoresis 27 (2006), Seiten 2111 - 2125 beschriebenen Migrationsmuster zu nutze machen. Wenn er mit Hilfe eines einfachen Diagramms (z.B. mit MS Excel) seine Messung in Form von m/z versus Migrationszeit plottet, werden ebenfalls die beschriebenen Linienmuster sichtbar. Durch Abzählen der Linien ist nun eine einfache Zuordnung der einzelnen Polypeptide möglich.

Auch andere Vorgehensweisen zur Zuordnung sind möglich. Grundsätzlich könnte der Fachmann auch die oben genannten Peptide als internen Standard verwenden, um seine CE-Messungen zuzuordnen.

**Tabelle 1**

| Masse (Da) | CE-T (min) |
|---|---|
| 858,39 | 23,24 |
| 884, 32 | 24,85 |
| 911,43 | 25,88 |
| 981,59 | 24,8 |
| 984,46 | 24.92, |
| 1013,37 | 25,17 |
| 1018,46 | 24,54 |
| 1040,47 | 25,05 |
| 1050,48 | 26,92 |
| 1058,48 | 24,89 |
| 1070,49 | 36,49 |
| 1071,49 | 21,43 |
| 1080,48 | 27,77 |
| 1080,5 | 25,69 |
| 1096,48 | 26,08 |
| 1100,5 | 37,04 |
| 1114,49 | 25,55 |
| 1128,49 | 25,65 |
| 1141,54 | 37,33 |
| 1143,52 | 36,97 |
| 1153,31 | 35,61 |
| 1157,54 | 37,44 |
| 1162,54 | 20,11 |
| 1173,53 | 37,49 |
| 1180,52 | 35,7 |
| 1182,55 | 28,27 |
| 1186,53 | 22,39 |
| 1191,52 | 36,18 |
| 1210,39 | 36,48 |
| 1211,54 | 25,82 |
| 1216,54 | 24,24 |
| 1217,53 | 35,78 |
| 1260,56 | 21,83 |
| 1262,46 | 38,23 |
| 1263,54 | 22,73 |
| 1265,59 | 27,09 |
| 1270,55 | 29,38 |
| 1276,4 | 35,92 |
| 1281,58 | 27,09 |
| 1299,58 | 22,38 |
| 1299,58 | 22,38 |
| 1312,55 | 29,77 |
| 1312,62 | 22,45 |
| 1324,59 | 28,7 |
| 1326,55 | 29,2 |
| 1337,62 | 38,2 |
| 1351,64 | 38,76 |
| 1353,66 | 25,63 |
| 1367,64 | 38,88 |
| 1378,61 | 28,82 |
| 1383,64 | 38,94 |
| 1396,62 | 26,67 |
| 1405,64 | 20,14 |
| 1407,66 | 37,23 |
| 1408,66 | 39,13 |
| 1422,68 | 28,14 |
| 1424,66 | 39,3 |
| 1438,66 | 30,2 |
| 1438,67 | 27,88 |
| 1439,66 | 29,82 |
| 1440,66 | 39,28 |
| 1442,63 | 27,63 |
| 1458,63 | 27,94 |
| 1470,68 | 21,08 |
| 1482,67 | 22,47 |
| 1485,67 | 23,77 |
| 1486,68 | 21,15 |
| 1491,74 | 39,83 |
| 1494,66 | 30,4 |
| 1496,68 | 30,38 |
| 1507,74 | 40,02 |
| 1508,68 | 29,33 |
| 1510,68 | 20,17 |
| 1513,44 | 36,79 |
| 1521,69 | 39,53 |
| 1523,74 | 40,66 |
| 1523,84 | 29,75 |
| 1525,48 | 37,17 |
| 526,69 | 23,92 |
| 154,97 | 39,49 |
| 1551,66 | 22,29 |
| 1552,5 | 37,22 |
| 1608,68 | 22,35 |
| 1608,73 | 30,93 |
| 1612,76 | 23,38 |
| 1624,55 | 37,73 |
| 1638,73 | 20,23 |
| 1640,58 | 23,24 |
| 1640,68 | 28,04 |
| 1654,78 | 23,13 |
| 1664,75 | 29,81 |
| 1669,69 | 21,47 |
| 1692,8 | 30,89 |
| 1697,74 | 30,88 |
| 1698,57 | 37,73 |
| 1716,77 | 28 |
| 1725,59 | 38,32 |
| 1732,77 | 28,17 |
| 1749,81 | 30,61 |
| 1750,78 | 23,83 |
| 1764,68 | 19,91 |
| 1765,81 | 31 |
| 1769,71 | 28,14 |
| 1793,88 | 32,37 |
| 1798,72 | 36,95 |
| 1809,88 | 32,3 |
| 1817,69 | 20,23 |
| 1818,83 | 30,95 |
| 1822,73 | 30,87 |
| 1823,99 | 24,4 |
| 1835,71 | 19,91 |
| 1837,8 | 30,56 |
| 1840,84 | 41,18 |
| 1847,89 | 43,67 |
| 1860,83 | 21,4 |
| 1880,9 | 43,91 |
| 1885,65 | 38,82 |
| 1892,86 | 24,33 |
| 1915,91 | 31,3 |
| 1916,77 | 20,32 |
| 1934,79 | 19,94 |
| 1942,84 | 30,96 |
| 1945 | 33,71 |
| 1962,88 | 31,81 |
| 1963,88 | 31,74 |
| 1996,79 | 20,98 |
| 2034,99 | 40,19 |
| 2039,13 | 21,78 |
| 2055,94 | 25,44 |
| 2058,94 | 23,15 |
| 2067,82 | 20,62 |
| 2070,92 | 25,4 |
| 2076,95 | 21,78 |
| 2080,94 | 20,2 |
| 2128,98 | 26,97 |
| 2132,91 | 25,83 |
| 2137,94 | 21,79 |
| 2156,97 | 22,22 |
| 2168,97 | 32,91 |
| 2189 | 26,89 |
| 2191,99 | 22,39 |
| 2194,97 | 20,17 |
| 2216,03 | 33,83 |
| 2226,99 | 26,28 |
| 2235,04 | 34,17 |
| 2248,99 | 25,99 |
| 2249,04 | 20,53 |
| 2257,87 | 35,93 |
| 2276,02 | 27,23 |
| 2277,01 | 27,23 |
| 2280,94 | 36,22 |
| 2289,04 | 33,59 |
| 2292,02 | 27,28 |
| 2308,02 | 27,34 |
| 2319,07 | 33,82 |
| 2323,04 | 22,36 |
| 2339 | 34.01 |
| 2367,06 | 27,63 |
| 2377,1 | 20,8 |
| 2385,05 | 33,95 |
| 2421 | 34,86 |
| 2446,09 | 28,37 |
| 2471,16 | 34,77 |
| 2483,12 | 27,57 |
| 2485,13 | 34.41 |
| 2544,13 | 28,26 |
| 2559,18 | 19,41 |
| 2570,19 | 42,56 |
| 2583,15 | 23,68 |
| 2584,23 | 35,18 |
| 2587,2 | 21,1 |
| 2596,23 | 34,9 |
| 2612,21 | 34,9 |
| 2639,29 | 21,42 |
| 2644,22 | 21,15 |
| 2654,19 | 23.92, |
| 2668,25 | 41.97 |
| 2748,79 | 36,38 |
| 2751,34 | 29,23 |
| 2756,27 | 35,24 |
| 2767,32 | 21,67 |
| 2802,82 | 36,34 |
| 2839,35 | 24,2 |
| 2907,35 | 35,96 |
| 2912,17 | 25,56 |
| 2926,3 | 22,22 |
| 2939,15 | 33,77 |
| 2946,21 | 34,98 |
| 2973,45 | 24,37 |
| 3001,43 | 35,4 |
| 3002,24 | 23,8 |
| 3011,39 | 29,75 |
| 3013,29 | 22.3 |
| 3035,19 | 42,02 |
| 3064,32 | 20,57 |
| 3081,42 | 29,83 |
| 3091,44 | 28,4 |
| 3108,45 | 31,28 |
| 3139,49 | 29,48 |
| 3149,46 | 31,25 |
| 3165,46 | 31,32 |
| 3178,43 | 30,3 |
| 3193,38 | 22,64 |
| 3209,41 | 22,67 |
| 3256,53 | 33,03 |
| 3281,43 | 36,09 |
| 3292,54 | 39,42 |
| 3318,55 | 30,99 |
| 3333,72 | 23,83 |
| 3334,54 | 31,02 |
| 3337,45 | 22,81 |
| 3350,55 | 31,02 |
| 3359,58 | 31,9 |
| 3363,54 | 30,22 |
| 3385,55 | 25,49 |
| 3401,6 | 25,47 |
| 3405,48 | 25,97 |
| 3426,31 | 27,7 |
| 3575,75 | 32,36 |
| 3657,67 | 40,71 |
| 3696,76 | 26,94 |
| 3718,72 | 32,48 |
| 3734,72 | 32,5 |
| 3831,81 | 28,48 |
| 3870,81 | 33,49 |
| 3871,79 | 27,57 |
| 3943,83 | 33,63 |
| 4002,62 | 20,66 |
| 4047,92 | 25,45 |
| 4078,81 | 33,14 |
| 4217,98 | 26,05 |
| 4251,98 | 28,76 |
| 4289,93 | 28,78 |
| 4368,9 | 20,21 |
| 4436,08 | 26,32 |
| 4467,96 | 29,12 |
| 4630 | 29,38 |
| 4863,16 | 26,74 |
| 8917,25 | 22,55 |

**Tabelle 2**

| Masse | CE-T | AUC | Normalkontrolle Häufigkeit | Normalkontrolle mean(mediam) Amp | Diabetes Häufigkeit | Diabetes mean(mediam) Amp | Bonferoni korrigierter p-Wert |
|---|---|---|---|---|---|---|---|
| 858,39 | 23,24 | 0,6586 | 0,83 | 2,33(2,34) | 0,63 | 2,28(2,25) | 0,0435652 |
| 884,32 | 24,85 | 0,6104 | 0,66 | 2,29(2,28) | 0,81 | 2,47(2,42) | 0,0263486 |
| 911,43 | 25,88 | 0,6586 | 0,87 | 2,71(2,72) | 0,72 | 2,51(2,61) | 0,0027488 |
| 981,59 | 24,8 | 0,6787 | 0,95 | 2,81(2,89) | 0,7 | 2,66(2,73) | 5,72E-12 |
| 984,46 | 24,92 | 0,6345 | 0,32 | 1,81(1,85) | 0,49 | 1,95(2,03) | 0,0018068 |
| 1013,37 | 25,17 | 0,6847 | 0,93 | 2,94(2,93) | 0,95 | 3,32(3,35) | 0,0430425 |
| 1018,46 | 24,54 | 0,6406 | 0,44 | 2,05(2,09) | 0,59 | 2,32(2,40) | 0,000435 |
| 1040,47 | 25,05 | 0,6867 | 0,8 | 2,37(2,41) | 0,51 | 2,32(2,38) | 0,0001277 |
| 1050,48 | 26,92 | 0,7068 | 0,97 | 2,81(2,84) | 0,8 | 2,57(2,62) | 1,471E-08 |
| 1058,48 | 24,89 | 0,7992 | 0,08 | 1,77(1,68) | 0,53 | 2,93(3,06) | 4,26E-16 |
| 1070,49 | 36,49 | 0,5904 | 0,52 | 2,24(2,27) | 0,27 | 2,06(2,07) | 4,881E-05 |
| 1071,49 | 21,43 | 0,6185 | 0,61 | 2,16(2,17) | 0,32 | 1,94(1,94) | 1,807E-06 |
| 1080,48 | 27,77 | 0,6827 | 0,66 | 2,19(2,22) | 0,29 | 2,10(2,23) | 7,221E-06 |
| 1080,5 | 25,69 | 0,6606 | 0,62 | 2,21(2,24) | 0,26 | 2,12(2,17) | 7,60E-12 |
| 1096,48 | 26,08 | 0,7711 | 0,98 | 3,78(3,80) | 0,81 | 3,26(3,49) | 3,02E-11 |
| 1100,5 | 37,04 | 0,6265 | 0,7 | 2,31(2,36) | 0,46 | 2,13(2,17) | 0,0004769 |
| 1114,49 | 25,55 | 0,7369 | 0,96 | 3,55(3,60) | 0,85 | 3,24(3,41) | 4,12E-06 |
| 1128,49 | 25,65 | 0,7008 | 0,8 | 2,44(2,47) | 0,5 | 2,20(2,27) | 6,13E-10 |
| 1141,52 | 24,51 | 0,6365 | 0,66 | 2,27(2,28) | 0,38 | 2,05(2,06) | 1,857E-06 |
| 1141,54 | 37,33 | 0,6647 | 0,75 | 2,52(2,57) | 0,48 | 2,32(2,26) | 2.385E-07 |
| 1143,52 | 36,97 | 0,6908 | 0,87 | 2,65(2,70) | 0,64 | 2,39(2,42) | 1,596E-09 |
| 1153,31 | 35,61 | 0,5683 | 0,67 | 2,55(2,58) | 0,69 | 2,57(2,62) | 0,0424232 |
| 1157,54 | 37,44 | 0,7269 | 0,95 | 3,21(3,27) | 0,82 | 2,88(2,92) | 7,29E-10 |
| 1162,54 | 20,11 | 0,5944 | 0,61 | 2,34(2,39) | 0,36 | 2,19(2,20) | 0,0136225 |
| 1173,53 | 37,49 | 0,6707 | 0,77 | 2,47(2,52) | 0,51 | 2,17(2,17) | 1,47E-11 |
| 1180,52 | 35,7 | 0,7088 | 0,79 | 2,78(2,83) | 0,39 | 2,40(2,49) | 6,26E-11 |
| 1182,55 | 28,27 | 0,7229 | 0,66 | 2,08(2,11) | 0,16 | 1,97(1,96) | 6,62E-16 |
| 1186,53 | 22,39 | 0,739 | 0,87 | 2,87(2,88) | 0,66 | 2,48(2,47) | 1.702E-09 |
| 1191,52 | 36,18 | 0,7068 | 0,83 | 2,65(2,72) | 0,48 | 2,33(2,35) | 2,08E-11 |
| 1210,39 | 36,48 | 0,6345 | 0,63 | 2,57(2,58) | 0,33 | 2,48(2,50) | 0,0131101 |
| 1211,54 | 25,82 | 0,6566 | 0,63 | 2,10(2,10) | 0,3 | 1,99(2,00) | 5,189E-07 |
| 1216,54 | 24,24 | 0,7088 | 0,87 | 3,14(3,15) | 0,7 | 2,77(2,82) | 0,0381734 |
| 1217,53 | 35.78 | 0,6747 | 0,76 | 3,49(3,61) | 0,41 | 3,17(3,29) | 5.698E-10 |
| 1260,56 | 21,83 | 0,6767 | 0,38 | 2,39(2,38) | 0,56 | 2,67(2,66) | 0,0302727 |
| 1262,46 | 38,23 | 0,6044 | 0,51 | 2,23(2,27) | 0,2 | 1,95(2,01) | 3,471 E-10 |
| 1263,54 | 22,73 | 0,6426 | 0,81 | 2,59(2,60) | 0,63 | 2,57(2,57) | 0,023065 |
| 1265,59 | 27,09 | 0,7771 | 0,95 | 3,78(3,80) | 0,67 | 3,47(3,46) | 1,39E-13 |
| 1270,55 | 29,38 | 0,6687 | 0,66 | 2,27(2,29) | 0,29 | 2,05(2,07) | 0,0001114 |
| 1276,4 | 35,92 | 0,5964 | 0,98 | 3,54(3,57) | 0,97 | 3,41(3,41) | 0,0023336 |
| 1281,58 | 27,09 | 0,6787 | 0,75 | 2,47(2,52) | 0,41 | 2,26(2,31) | 8,713E-08 |
| 1297,58 | 27,36 | 0,745 | 0,86 | 3,17(3,21) | 0,41 | 3,05(3,09) | 1,80E-16 |
| 1299,58 | 22,38 | 0,7129 | 0,84 | 2,51(2,54) | 0,48 | 2,41(2,47) | 5.19E-10 |
| 1312,55 | 29,77 | 0,5462 | 0,96 | 3,15(3,20) | 0,95 | 3,08(3,09) | 2.657E-07 |
| 1312,62 | 22,45 | 0,5803 | 0,74 | 2,71(2,76) | 0,79 | 2,80(2,81) | 0,018991 |
| 1324,59 | 28,7 | 0,6124 | 0,45 | 2,50(2,52) | 0,66 | 2,63(2,71) | 0,0001963 |
| 1326,55 | 29,2 | 0,6687 | 0,8 | 2,39(2,42) | 0,49 | 2,16(2,17) | 7,645E-08 |
| 1337,62 | 38,2 | 0,6767 | 0,65 | 2,34(2,37) | 0,3 | 1,89(1,90) | 2.92E-17 |
| 1351,64 | 38,76 | 0,7008 | 0,8 | 2,57(2,60) | 0,49 | 2,27(2,28) | 2,01E-12 |
| 1353,66 | 25,63 | 0,7129 | 0,91 | 2,70(2,72) | 0,79 | 2,40(2,47) | 6,629E-09 |
| 1367,64 | 38,88 | 0,747 | 0,94 | 2,98(3,03) | 0,78 | 2,61(2,66) | 3,73E-13 |
| 1378,61 | 28,82 | 0,7369 | 0,99 | 3,57(3,57) | 0,97 | 3,33(3,35) | 4,196E-05 |
| 1383,64 | 38,94 | 0,6225 | 0,54 | 2,27(2,29) | 0,23 | 1,94(1,96) | 4,281E-06 |
| 1396,62 | 26,67 | 0,5522 | 0,61 | 2,13(2,14) | 0,47 | 2,02(2,03) | 0,0002984 |
| 1405,64 | 20,14 | 0,6406 | 0,59 | 2,44(2,48) | 0,26 | 2,06(2,08) | 3,016E-09 |
| 1407,66 | 37,23 | 0,6968 | 0,73 | 2,47(2,51) | 0,32 | 2,09(2,11) | 2,03E-14 |
| 1408,66 | 39,13 | 0,6888 | 0,85 | 2,89(2,94) | 0,66 | 2,58(2,63) | 2,051 E-09 |
| 1422,68 | 28,14 | 0,7088 | 0,83 | 3,47(3,49) | 0,52 | 3,52(3,51) | 9.995E-08 |
| 1424,66 | 39,3 | 0,6908 | 0,93 | 3,47(3,53) | 0,82 | 3,11(3,21) | 5,458E-09 |
| 1438,66 | 30,2 | 0,5884 | 0,51 | 2,19(2,23) | 0,26 | 2,14(2,22) | 7,114E-06 |
| 1438,67 | 27,88 | 0,747 | 0,97 | 3,54(3,55) | 0,9 | 3,27(3,41) | 0,0006262 |
| 1439,66 | 29,82 | 0,6365 | 0,56 | 2,22(2,22) | 0,64 | 2,37(2,34) | 0,000113 |
| 1440,66 | 39,28 | 0,6647 | 0,8 | 2,76(2,80) | 0,52 | 2,58(2,62) | 7,215E-09 |
| 1442,63 | 27,63 | 0,739 | 0,2 | 1,93(1,97) | 0,53 | 2,48(2,49) | 4,70E-10 |
| 1458,63 | 27,94 | 0,6727 | 0,38 | 2,29(2,33) | 0,55 | 2,69(2,75) | 0,0033535 |
| 1470,68 | 21,08 | 0,6064 | 0,52 | 2,09(2,06) | 0,24 | 1,84(1,86) | 1,93E-06 |
| 1482,67 | 22,47 | 0,5723 | 0,56 | 2,67(2,70) | 0,41 | 2,40(2,39) | 0,0091356 |
| 1485,67 | 23,77 | 0,745 | 0,98 | 3,11(3,14) | 0,88 | 2,84(2,89) | 1,101E-07 |
| 486,68 | 21,15 | 0,6807 | 0,8 | 2,57(2,57) | 0,5 | 2,28(2,29) | 1,71E-10 |
| 91,74 | 39,83 | 0,7108 | 0,88 | 2,83(2,87) | 0,65 | 2,49(2,52) | 3,34E-13 |
| 194,66 | 30,4 | 0,6586 | 0,86 | 2,31(2,32) | 0,68 | 2,26(2,26) | 0,0279139 |
| 1496,68 | 30,38 | 0,6165 | 0,63 | 2,25(2,24) | 0,37 | 2,07(2,05) | 0,0017352 |
| 1507,74 | 40,02 | 0,7149 | 0,93 | 3,58(3,61) | 0,89 | 3,21(3,23) | 5,548E-08 |
| 1508,68 | 29,33 | 0,7108 | 0,97 | 3,49(3,52) | 0,88 | 3,27(3,40) | 0,0005901 |
| 1510,68 | 20,17 | 0,6667 | 0,35 | 2,25(2,27) | 0,53 | 2,54(2,58) | 0,0001918 |
| 1513,44 | 36,79 | 0,6225 | 0,59 | 2,41(2,40) | 0,31 | 2,26(2,16) | 2,318E-05 |
| 121,69 | 30,53 | 0,5964 | 0,55 | 2,11(2,13) | 0,32 | 1,98(1,86) | 0,0091176 |
| 1523,74 | 40,66 | 0,7309 | 0,87 | 4,18(4,21) | 0,89 | 3,83(3,86) | 0,0052433 |
| 1523,84 | 29,75 | 0,5743 | 0,68 | 3,32(3,39) | 0,53 | 3,18(3,25) | 0,0003408 |
| 1525,48 | 37,17 | 0,6245 | 0,98 | 3,30(3,32) | 0,94 | 3,19(3,19) | 0,0199067 |
| 1526,69 | 23,92 | 0,6486 | 0,72 | 2,18(2,18) | 0,49 | 2,01(2,01) | 3,234E-05 |
| 1549,7 | 39,49 | 0,6466 | 0,7 | 2,33(2,34) | 0,44 | 2,33(2,34) | 0,0139286 |
| 1551,66 | 22,29 | 0,6124 | 0,64 | 2,31(2,34) | 0,41 | 2,14(2,18) | 7,338E-07 |
| 1552,5 | 37,22 | 0,7028 | 0,98 | 3,31(3,32) | 0,91 | 3,09(3,11) | 3,707E-05 |
| 1608,68 | 22,35 | 0,6205 | 0,68 | 2,31(2,32) | 0,49 | 2,11(2,15) | 2,061E-05 |
| 1608,73 | 30,93 | 0 6084 | 0,81 | 2,70(2,66) | 0,64 | 2,45(2,39) | 0,0009389 |
| 1612,76 | 23,38 | 0,6245 | 0,58 | 2,23(2,24) | 0,31 | 2,06(2,08) | 0,000304 |
| 1624,55 | 37,73 | 0,6586 | 0,97 | 3,05(3,06) | 0,93 | 2,91(2,90) | 0,0004413 |
| 1638,73 | 20,23 | 0,6245 | 0,63 | 2,68(2,71) | 0,74 | 2,94(2,94) | 0,0178203 |
| 1640,58 | 23,24 | 0,6305 | 0,91 | 3,60(3,62) | 0,85 | 3,39(3,44) | 0,0436627 |
| 1640,68 | 28,04 | 0,6747 | 0,33 | 1,85(1,87) | 0,52 | 2,14(2,17) | 0,0019075 |
| 1654,78 | 23,13 | 0,7149 | 0,25 | 2,08(2,08) | 0,59 | 2,52(2,52) | 1,37E-10 |
| 1664,75 | 29,81 | 0,6325 | 0.97 | 2,84(2,86) | 0,87 | 2,77(2,82) | 0,0003779 |
| 1669,69 | 21,47 | 0,7149 | 0,58 | 2,24(2,24) | 0,75 | 2,71(2,70) | 3,742E-06 |
| 1692,8 | 30,89 | 0,753 | 0,96 | 3,00(3,04) | 0,67 | 2,65(2,69) | 1,43E-13 |
| 1697,74 | 30,88 | 0,5863 | 0,97 | 3,03(3,03) | 0,94 | 2,98(3,00) | 0,0011862 |
| 1698,57 | 37,73 | 0,5763 | 0,61 | 2,79(2,91) | 0,39 | 2,41(2,41) | 0,0036709 |
| 1716,77 | 28 | 0,7149 | 0,89 | 2,72(2,76) | 0,6 | 2,43(2,52) | 3,30E-12 |
| 1725,59 | 38,32 | 0,6205 | 0,98 | 3,24(3,25) | 0,9 | 3,09(3,11) | 9,115E-05 |
| 1732,77 | 28,17 | 0,7329 | 0,92 | 3,44(3,49) | 0,68 | 3,27(3,32) | 2.937E-08 |
| 1749,81 | 30,61 | 0,6024 | 0,8 | 2,55(2,53) | 0,64 | 2,35(2,38) | 5,129E-06 |
| 1750,78 | 23,83 | 0,749 | 0,95 | 2,98(3,06) | 0,71 | 2,60(2,64) | 5,45E-12 |
| 1764,68 | 19,91 | 0,6024 | 0,57 | 2,53(2,55) | 0,3 | 2,33(2,33) | 8,56E-05 |
| 1765,81 | 31 | 0,6707 | 0,95 | 3,23(3,26) | 0,82 | 3,04(3,10) | 2,384E-05 |
| 1769,71 | 28,14 | 0,6526 | 0,59 | 2,14(2,17) | 0,73 | 2,43(2,49) | 0,0367372 |
| 1793,88 | 32,37 | 0,6506 | 0,58 | 2,14(2,14) | 0,84 | 2,37(2,43) | 1,598E-06 |
| 1798,72 | 36,95 | 0,6446 | 0,62 | 2,26(2,35) | 0,27 | 2,02(2,03) | 5,116E-07 |
| 1809,88 | 32,3 | 0,7369 | 0,19 | 1,85(1,87) | 0,6 | 2,19(2,22) | 7,12E-17 |
| 1817,6 | 20,23 | 0,6185 | 0,95 | 3,35(3,43) | 0,81 | 3,20(3,24) | 7,771E-06 |
| 1818,83 | 30,95 | 0,7731 | 0,69 | 2,30(2,33) | 0,82 | 3,02(3,20) | 4,883E-06 |
| 1822,73 | 30,87 | 0,6787 | 0,74 | 2,48(2,51) | 0,41 | 2,50(2,57) | 4,31E-09 |
| 1823,99 | 24,4 | 0,5341 | 0,62 | 2,69(2,70) | 0,51 | 2,54(2,52) | 0,0282935 |
| 1835,71 | 19,91 | 0,6084 | 0,77 | 2,87(2,94) | 0,55 | 2,67(2,68) | 2,228E-05 |
| 1837,8 | 30,56 | 0,6667 | 0,57 | 2,44(2,46) | 0,16 | 2,33(2,37) | 2,44E-15 |
| 1840,84 | 41,18 | 0,6205 | 0,62 | 2,38(2,42) | 0,35 | 2,15(2,32) | 2,254E-07 |
| 1847,89 | 43,67 | 0,6205 | 0,49 | 2,58(2,66) | 0,14 | 2,30(2,35) | 2,45E-12 |
| 1860,83 | 21,4 | 0,7631 | 0,89 | 2,87(2,93) | 0,44 | 2,69(2,77) | 1,06E-21 |
| 1880,9 | 43,91 | 0,6145 | 0,67 | 2,97(3,09) | 0,45 | 2,60(2,72) | 2,75E-10 |
| 1885,6 | 38,82 | 0,6546 | 0,75 | 2,14(2,20) | 0,53 | 2,08(2,10) | 5,025E-05 |
| 1892,86 | 24,33 | 0,6707 | 0,25 | 2,21(2,18) | 0,53 | 2,32(2,28) | 4,178E-07 |
| 1915,91 | 31,3 | 0,5924 | 0,52 | 2,07(2,10) | 0,26 | 1,93(1,97) | 1,28E-11 |
| 1916,7 | 20,32 | 0,6365 | 0,93 | 3,15(3,23) | 0,78 | 3,01(3,07) | 7,495E-05 |
| 1934,79 | 19,94 | 0,6185 | 0,72 | 2,75(2,83) | 0,52 | 2,55(2,62) | 0,0001583 |
| 1942,84 | 30,96 | 0,6325 | 0,68 | 2,07(2,07) | 0,42 | 1,94(1,95) | 7,525E-09 |
| 1945 | 33,71 | 0,5703 | 0,65 | 2,25(2,31) | 0,87 | 2,32(2,37) | 0,0050419 |
| 1962,88 | 31,81 | 0,6546 | 0,34 | 2,36(2,43) | 0,64 | 2,34(2,44) | 0,0004143 |
| 1963,88 | 31, 74 | 0,6526 | 0,64 | 2,31(2,33) | 0,32 | 2,29(2,28) | 3,665E-06 |
| 1996,79 | 20,98 | 0,6205 | 0,83 | 2,84(2,92) | 0,63 | 2,66(2,68) | 3.313E-05 |
| 2034,99 | 40,19 | 0,5803 | 0,49 | 2,11(2,20) | 0,23 | 1,89(1,86) | 6,53E-11 |
| 2039,13 | 21,78 | 0,6185 | 0,74 | 2,97(3,02) | 0,57 | 2,76(2,96) | 6,134E-05 |
| 2055,94 | 25,44 | 0,6606 | 0,98 | 2,91(2,96) | 0,9 | 2,85(2,91) | 0,0340338 |
| 2058,94 | 23,15 | 0,6807 | 0,83 | 2,51(2,57) | 0,49 | 2,30(2,38) | 9,04E-12 |
| 2067,82 | 20,62 | 0,6365 | 0,92 | 3,08(3,17) | 0,75 | 2,90(2,98) | 1,957E-06 |
| 2070,92 | 25,4 | 0,747 | 0,96 | 2,99(3,04) | 0,84 | 2,62(2,67) | 1,033E-09 |
| 2076,95 | 21,78 | 0,6546 | 0,71 | 2,92(2,98) | 0,43 | 2,44(2,41) | 3,20E-12 |
| 2080,94 | 20,2 | 0,6747 | 0,81 | 2,84(2,90) | 0,59 | 2,58(2,62) | 0,0002311 |
| 2128,98 | 26,97 | 0,7028 | 0,69 | 2,14(2,16) | 0,29 | 1,81(1,84) | 1,95E-16 |
| 2132,91 | 25,83 | 0,6667 | 0,57 | 2,31(2,33) | 0,69 | 2,69(2,72) | 0,0073888 |
| 2137,94 | 21,79 | 0,6426 | 0,95 | 2,84(2,87) | 0,82 | 2,74(2,77) | 0,027956 |
| 2156,97 | 22,22 | 0,753 | 0,96 | 3,11(3,15) | 0,71 | 2,72(2,80) | 2,91E-14 |
| 2168,97 | 32,91 | 0,5361 | 0,71 | 2,74(2,81) | 0,9 | 2,82(2,86) | 0,0011953 |
| 2189 | 26,89 | 0,6586 | 0,93 | 2,95(2,98) | 0,8 | 2,99(3,00) | 0,014082 |
| 2191,99 | 22,39 | 0,7189 | 0,82 | 2,70(2,73) | 0,51 | 2,24(2,25) | 1.43E-14 |
| 2194,97 | 20,17 | 0,6365 | 0,58 | 2,52(2,56) | 0,24 | 2,26(2,25) | 4,695E-09 |
| 2216,03 | 33,83 | 0,7269 | 0,95 | 2,65(2,69) | 0,87 | 2,24(2,22) | 1,569E-09 |
| 2226,99 | 26,28 | 0,747 | 0,91 | 4,22(4,21) | 0,63 | 3,90(3,97) | 4,09E-11 |
| 2235,04 | 34,17 | 0,6888 | 0,88 | 2,89(2,93) | 0,71 | 2,49(2,49) | 3,61E-11 |
| 2248,99 | 25,99 | 0,5783 | 0,61 | 4,22(4,23) | 0,88 | 4,20(4,23) | 4,206E-05 |
| 2249,04 | 20,53 | 0,6145 | 0,68 | 2,59(2,65) | 0,42 | 2,36(2,36) | 4,476E-07 |
| 2257,87 | 35,93 | 0,6446 | 0,64 | 2,93(2,93) | 0,32 | 2,41(2,52) | 6,77E-14 |
| 2276,02 | 27,23 | 0,757 | 0,85 | 3,60(3,68) | 0,41 | 3,34(3,46) | 8,24E-17 |
| 2277,01 | 27,23 | 0,7048 | 0,29 | 2,86(3,23) | 0,64 | 3,08(3,20) | 3,207E-05 |
| 2280,94 | 36,22 | 0,5984 | 0,54 | 2,41(2,45) | 0,24 | 2,07(2,07) | 9,57E-10 |
| 2289,04 | 33,59 | 0,6044 | 0,56 | 2,21(2,26) | 0,3 | 1,85(1,86) | 6.98E-10 |
| 2292,02 | 27,28 | 0,747 | 1 | 3,68(3,71) | 0,98 | 3,32(3,39) | 1,419E-09 |
| 2308,02 | 27,34 | 0,6867 | 0,74 | 2,36(2,43) | 0,37 | 2,02(2,05) | 1,92E-11 |
| 2319,07 | 33,82 | 0,6024 | 0,51 | 2,93(2,95) | 0,53 | 2,87(2,91) | 0,0051152 |
| 2323,04 | 22,36 | 0,6486 | 0,81 | 2,52(2,54) | 0,6 | 2,44(2,49) | 0,0001244 |
| 2339 | 34,01 | 0,7631 | 0,94 | 2,91(2,94) | 0,76 | 2,42(2,50) | 1,23E-11 |
| 2367,06 | 27,63 | 0,7169 | 0,18 | 1,76(1,75) | 0,5 | 2,07(2,10) | 0,0105485 |
| 2377,1 | 20,8 | 0,7229 | 0,93 | 3,22(3,32) | 0,78 | 2,83(2,87) | 3,352E-10 |
| 2385,05 | 33,95 | 0,6426 | 0,93 | 2,88(2,95) | 0,85 | 2,75(2,84) | 0,0005018 |
| 2421 | 34,86 | 0,6606 | 0,58 | 1,96(1,99) | 0,19 | 1,67(1,77) | 9.64E-16 |
| 2446,09 | 28,37 | 0,6466 | 0,75 | 2,36(2,39) | 0,54 | 2,14(2,18) | 2.941E-06 |
| 2471,16 | 34,77 | 0,6767 | 0,93 | 2,65(2,71) | 0,81 | 2,36(2,43) | 2,143E-06 |
| 2483,12 | 27,57 | 0,7349 | 0,91 | 2,72(2,80) | 0,71 | 2,34(2,41) | 3,44E-13 |
| 2485,13 | 34,41 | 0,5924 | 0,61 | 2,22(2,06) | 0,35 | 1,78(1,80) | 2.10E-12 |
| 2525,2 | 27,74 | 0,6185 | 0,77 | 2,92(3,00) | 0,65 | 2,70(2,80) | 0,0031028 |
| 2544,13 | 28,26 | 0,5843 | 0,62 | 2,19(2,25) | 0,49 | 2,22(2,25) | 0,0162953 |
| 2559,18 | 19,41 | 0,6627 | 0,41 | 2,58(2,63) | 0,58 | 3,04(3,02) | 0,0386686 |
| 2570,1 | 42,56 | 0,5924 | 0,88 | 3,59(3,68) | 0,82 | 3,44(3,48) | 0,0252135 |
| 2583,15 | 23,68 | 0,6285 | 0,61 | 2,29(2,29) | 0,34 | 2,27(2,32) | 0,0001382 |
| 2584,23 | 35,18 | 0,6767 | 0,92 | 2,95(3,00) | 0,86 | 2,64(2,72) | 1,116E-07 |
| 2587,2 | 21,11 | 0,6667 | 0,64 | 2,72(2,79) | 0,28 | 2,48(2,53) | 2,24E-16 |
| 2596,23 | 34,9 | 0,6084 | 0,53 | 1,89(1,92) | 0,21 | 1,62(1,69) | 4,67E-11 |
| 2612,21 | 34,9 | 0,6225 | 0,73 | 2,74(2,76) | 0,68 | 2,43(2,50) | 0,0471086 |
| 2639,29 | 21,42 | 0,6265 | 0,73 | 2,54(2,60) | 0,56 | 2,51(2,53) | 7.298E-09 |
| 2644,22 | 21,15 | 0,6325 | 0,64 | 2,62(2,62) | 0,35 | 2,32(2,33) | 1,59E-12 |
| 2654,19 | 23,92 | 0,6345 | 0,89 | 2,39(2,42) | 0,76 | 2,33(2,40) | 0,0103611 |
| 2668,25 | 41,97 | 0,6406 | 0,74 | 2,57(2,66) | 0,53 | 2,42(2,54) | 8.044E-07 |
| 2748,79 | 36,38 | 0,6064 | 0,51 | 1,86(1,88) | 0,22 | 1,72(1,72) | 0,0003881 |
| 2751,34 | 29,23 | 0,5783 | 0,5 | 2,51(2,58) | 0,27 | 2,30(2,42) | 6,60E-13 |
| 2756,27 | 35,24 | 0,6426 | 0,77 | 2,36(2,37) | 0,6 | 2,07(2,08) | 0,000661 |
| 2767,32 | 21,67 | 0,6466 | 0,71 | 2,58(2,64) | 0,47 | 2,39(2,43) | 1,54E-16 |
| 2802,82 | 36,34 | 0,6365 | 0,5 | 2,17(2,16) | 0,11 | 1,78(1,83) | 3,46E-24 |
| 2839,35 | 24,2 | 0,5763 | 0,56 | 2,96(3,10) | 0,43 | 2,49(2,74) | 0,000138 |
| 2907,35 | 35,96 | 0,6044 | 0,78 | 2,38(2,42) | 0,62 | 2,16(2,17) | 0,0275969 |
| 2912,17 | 25,56 | 0,7189 | 0,98 | 2,97(3,00) | 0,89 | 2,69(2,76) | 2.093E-07 |
| 2926,3 | 22,22 | 0,6024 | 0,65 | 2,63(2,64) | 0,45 | 2,51(2,55) | 0,0054093 |
| 2939,15 | 33,77 | 0,6004 | 0,63 | 2,33(2,38) | 0,44 | 2,05(2,10) | 5.882E-06 |
| 2946,21 | 34,98 | 0,6165 | 0,42 | 1,89(1,84) | 0,61 | 2,05(2,14) | 2.307E-05 |
| 2973,45 | 24,37 | 0,6145 | 0,75 | 2,68(2,71) | 0,59 | 2,59(2,57) | 3,743E-07 |
| 3001,43 | 35,4 | 0,6446 | 0,83 | 3,87(3,95) | 0,77 | 3,61(3,75) | 0,0363329 |
| 3002,24 | 23,81 | 0,6466 | 0,59 | 2,03(2,04) | 0,26 | 1,83(1,88) | 7,23E-10 |
| 3011,39 | 2975 | 0,6867 | 0,95 | 3,31(3,36) | 0,91 | 3,14(3,17) | 0,0003048 |
| 3013,29 | 22,3 | 0,7229 | 0,86 | 3,75(3,82) | 0,69 | 3,16(3,35) | 8,80E-11 |
| 3035,19 | 42,02 | 0,5643 | 0,68 | 2,62(2,70) | 0,55 | 2,36(2,43) | 0,0066459 |
| 3064,32 | 20,57 | 0,6345 | 0,6 | 2,70(2,74) | 0,31 | 2,50(2,53) | 7,237E-07 |
| 3081,42 | 29,83 | 0,5984 | 0,57 | 2,49(2,59) | 0,31 | 2,31(2,38) | 2,315E-06 |
| 3091,44 | 28,4 | 0,7048 | 0,8 | 2,75(2,79) | 0,66 | 2,39(2,45) | 4,189E-08 |
| 3108,45 | 31,28 | 0,6566 | 0,95 | 2,73(2,78) | 0,86 | 2,60(2,61) | 0,0016437 |
| 3139,49 | 29,48 | 0,5743 | 0,7 | 2,94(2,99) | 0,59 | 2,83(2,81) | 0,0458493 |
| 3149,46 | 31,25 | 0,6667 | 0,89 | 2,79(2,85) | 0,78 | 2,59(2,60) | 7,125E-05 |
| 3165,46 | 31,32 | 0,6526 | 0,85 | 2,54(2,58) | 0,74 | 2,32(2,32) | 0,005123 |
| 3178,43 | 30,3 | 0,7309 | 0,4 | 2,06(2,10) | 0,65 | 2,58(2,62) | 1,023E-08 |
| 3193,38 | 22,64 | 0,6647 | 0,7 | 3,17(3,23) | 0,38 | 2,81(2,89) | 3,766E-08 |
| 3209,41 | 22,67 | 0,7129 | 0,96 | 3,77(3,79) | 0,86 | 3,55(3,63) | 1,663E-05 |
| 3256,53 | 33,03 | 0,6988 | 0,7 | 2,78(2,80) | 0,3 | 2,63(2,61) | 1,121E-06 |
| 3281,43 | 36,09 | 0,6406 | 0,96 | 3,17(3,23) | 0,88 | 3,08(3,13) | 0,0046604 |
| 3292,54 | 39,42 | 0,6586 | 0,9 | 3,62(3,69) | 0,74 | 3,43(3,49) | 1,357E-09 |
| 3318,55 | 30,99 | 0,6104 | 0,71 | 2,27(2,30) | 0,49 | 2,09(2,10) | 9,729E-05 |
| 3333,72 | 23,83 | 0,747 | 0,18 | 2,20(2,26) | 0,55 | 2,80(2,89) | 5,19E-10 |
| 3334,54 | 31,02 | 0,5482 | 0,48 | 2,47(2,56) | 0,33 | 2,17(2,24) | 4,246E-05 |
| 3337,45 | 22,81 | 0,6627 | 0,68 | 2,89(2,94) | 0,36 | 2,47(2,54) | 4,37E-18 |
| 3350,55 | 31,02 | 0,5924 | 0,58 | 2,24(2,25) | 0,44 | 1,95(2,00) | 0,0044159 |
| 3359,58 | 31,9 | 0,6968 | 0,94 | 3,23(3,28) | 0,91 | 2,93(2,96) | 0,0059278 |
| 3363,54 | 30,22 | 0,747 | 0,39 | 2,07(2,08) | 0,65 | 2,51(2,50) | 4,15E-08 |
| 3385,55 | 25,49 | 0,6486 | 0,78 | 3,53(3,59) | 0,56 | 3,25(3,35) | 1,93E-13 |
| 3401,6 | 25,47 | 0,6325 | 0,85 | 2,98(3,06) | 0,76 | 2,82(2,87) | 4.667E-08 |
| 3405,48 | 25,97 | 0,5482 | 0,53 | 3,41(3,50) | 0,42 | 3,44(3,51) | 0,0079805 |
| 3426,31 | 27,7 | 0,6225 | 0,44 | 1,94(2,00) | 0,6 | 2,11(2,15) | 0,002223 |
| 3575,75 | 32,36 | 0,6707 | 0,31 | 1,94(1,94) | 0,58 | 2,20(2,17) | 1,274E-05 |
| 3657,67 | 40,71 | 0,6124 | 0,7 | 2,93(3,00) | 0,55 | 2,67(2,71) | 2,29E-06 |
| 3696,76 | 26,94 | 0,5904 | 0,6 | 2,37(2,43) | 0,38 | 2,25(2,27) | 2.447E-06 |
| 3718,72 | 32,48 | 0,6988 | 0,92 | 3,05(3,11) | 0,81 | 2,78(2,82) | 1,69E-06 |
| 3734,72 | 32,5 | 0,6586 | 0,88 | 2,83(2,91) | 0,83 | 2,56(2,57) | 1,702E-05 |
| 3831,81 | 28,48 | 0,6205 | 0,73 | 2,90(3,00) | 0,55 | 2,74(2,78) | 0,0031118 |
| 3870,81 | 33,49 | 0,5643 | 0,57 | 2,20(2,23) | 0,37 | 1,97(2,04) | 0,0003836 |
| 3871,79 | 27,57 | 0,5823 | 0,6 | 2,48(2,55) | 0,41 | 2,40(2,48) | 0,0280454 |
| 3943,83 | 33,63 | 0,6165 | 0,78 | 2,39(2,44) | 0,72 | 2,13(2,14) | 0,0186637 |
| 4002,62 | 20,66 | 0,5502 | 0,71 | 2,95(3,07) | 0,63 | 2,83(2,87) | 0,0051078 |
| 4047,92 | 25,45 | 0,6165 | 0,42 | 2,18(2,21) | 0,59 | 2,33(2,30) | 0,0148071 |
| 4078,81 | 33,14 | 0,7289 | 0,42 | 2,00(2,00) | 0,66 | 2,41(2,38) | 1,67E-05 |
| 4217,98 | 26,05 | 0,6205 | 0,68 | 3,49(3,59) | 0,51 | 3,31(3,37) | 1,7E-05 |
| 4251,98 | 28,76 | 0,6466 | 0,78 | 3,00(3,05) | 0,66 | 2,74(2,80) | 1,238E-06 |
| 4289,93 | 28,78 | 0,6546 | 0,83 | 3,67(3,70) | 0,63 | 3,54(3,60) | 0,0018768 |
| 4368,9 | 20,21 | 0,5643 | 0,68 | 3,10(3,13) | 0,6 | 3,01(3,05) | 0,0436271 |
| 4436,08 | 26,32 | 0,5843 | 0,58 | 3,29(3,31) | 0,42 | 3,12(3,13) | 0,0008656 |
| 4467,96 | 29,12 | 0,5361 | 0,53 | 2,62(2,71) | 0,43 | 2,43(2,52) | 0,0015007 |
| 4630 | 29,38 | 0,5984 | 0,6 | 2,73(2,78) | 0,39 | 2,58(2,66) | 3,49E-06 |
| 4863,16 | 26,74 | 0,5884 | 0,67 | 2,72(2,88) | 0,57 | 2,69(2,79) | 0,0004877 |
| 8917,25 | 22,55 | 0,6245 | 0,37 | 2,43(2,46) | 0,55 | 2,55(2,47) | 0,0004797 |

**Tabelle 3**

| Masse | CE-T | Sequenz | Proteinname | Protein coverage | Swissprot Name |
|---|---|---|---|---|---|
| 858,39 | 23,24 | | | | |
| 884,32 | 24,85 | | | | |
| 911,43 | 25,88 | | | | |
| 981,59 | 24,8 | VLNLGPITR | Uromodulin | 598-606 | UROM_HUMAN |
| 984,46 | 24,92 | | | | |
| 1013,37 | 25,17 | | | | |
| 1018,46 | 24,54 | | | | |
| 1040,47 | 25,05 | SPhGPDGKTGPPh | Collagen alpha-1 (I) chain | 546-556 | |
| 1050,48 | 26,92 | MGPRGPPhGPPhG | Collagen alpha-1 (I) chain | 217-227 | CO1A1_HUMAN |
| 1058,48 | 24,89 | | | | |
| 1070,49 | 36,49 | | | | |
| 1071,49 | 21,43 | | | | |
| 1080,48 | 27,77 | | | | |
| 1080,5 | 25,69 | | | | |
| 1096,48 | 26,08 | APhGDRGEPhGPPh | Collagen alpha-1 (I) chain | 798-808 | CO1A1_HUMAN |
| 1100,5 | 37,04 | | | | |
| 1114,49 | 25,55 | | | | |
| 1128,49 | 25,65 | | | | |
| 1141,52 | 24,51 | | | | |
| 1141,54 | 37,33 | GPPhGPhPGPPGPPS | Collagen alpha-1 (I) chain | 1181-1193 | CO1A1_HUMAN |
| 1143,52 | 36,97 | | | | |
| 1153,31 | 35,61 | | | | |
| 1157,54 | 37,44 | GPPGPhPhGPhPGPPS | Collagen alpha-1 (I) chain | 1181-1193 | CO1A1_HUMAN |
| 1162,54 | 20,11 | | | | |
| 1173,53 | 37,49 | | | | |
| 1180,52 | 35,7 | | | | |
| 1182,55 | 28,27 | | | | |
| 1186,53 | 22,39 | DDGEAGKPhGRPhG | Collagen alpha-1 (I) chain | 231-242 | CO1A1_HUMAN |
| 1191,52 | 36,18 | | | | |
| 1210,39 | 36,48 | | | | |
| 1211,54 | 25,82 | | | | |
| 1216,54 | 24,24 | | | | |
| 1217,53 | 35,78 | | | | |
| 1260,56 | 21,83 | | | | |
| 1262,46 | 38,23 | | | | |
| 1263,54 | 22,73 | | | | |
| 1265,59 | 27,09 | SPhGPDGKTGPPhGPA | Collagen alpha-1 (I) chain | 546-559 | CO1A1_HUMAN |
| 1270,55 | 29,38 | | | | |
| 1276,4 | 35,92 | | | | |
| 1281,58 | 27,09 | | | | |
| 1297,58 | 27,36 | SPhGSPhGPDGKTGPPh | Collagen alpha-1 (I) chain | 543-556 | CO1A1_HUMAN |
| 1299,58 | 22,38 | | | | |
| 1312,55 | 29,77 | | | | |
| 1312,62 | 22,45 | | | | |
| 1324,59 | 28,7 | | | | |
| 1326,55 | 29,2 | SPhGGPhGSDGKPhGPPhG | Collagen alpha-1 (III) chain | 541-555 | CO3A1_HUMAN |
| 1337,62 | 38,2 | | | | |
| 1351,64 | 38,76 | | | | |
| 1353,66 | 25,63 | | | | |
| 1367,64 | 38,88 | | | | |
| 1378,61 | 28,82 | APhGEDGRPhGPPhGPQ | Collagen alpha-1 (II) chain | 511-524 | CO2A1_HUMAN |
| 1383,64 | 38,94 | | | | |
| 1396,62 | 26,67 | | | | |
| 1405,64 | 20,14 | DGPPhGRDGQPhGHKG | Collagen alpha-2 (I) chain | 933-946 | CO1A2_HUMAN |
| 1407,66 | 37,23 | | | | |
| 1408,66 | 39,13 | | | | |
| 1422,68 | 28,14 | | | | |
| 1424,66 | 39,3 | GLPGPPhGPPhGSFLSN | Collagen alpha-1 (XVII) chain | 885-899 | COHA1_HUMAN |
| 1438,66 | 30,2 | SLPhGTGGPPhGENGKPhG | Collagen alpha-1 (III) chain | 642-657 | CO3A1_HUMAN |
| 1438,67 | 27,88 | | | | |
| 1439,66 | 29,82 | TIDEKGTEAAGAMF | Alpha-1-antitrypsin | 328-341 | A1AT_HUMAN |
| 1440,66 | 39,28 | | | | |
| 1442,63 | 27,63 | | | | |
| 1458,63 | 27,94 | SPhGENGAPhGQMoxGPRG | Collagen alpha-1 (I) chain | 291-305 | CO1A1_HUMAN |
| 1470,68 | 21,08 | | | | |
| 1482,67 | 22,47 | | | | |
| 1485,67 | 23,77 | DGQPhGAKGEPhGDAGAK | Collagen alpha-1 (I) chain | 820-835 | CO1A1_HUMAN |
| 1486,68 | 21,15 | | | | |
| 1491,74 | 39,83 | VGPPhGPhPGPPGPPGPPS | Collagen alha-1 (I) chain | 1174-1190 | CO1A1_HUMAN |
| 1494,66 | 30,4 | | | | |
| 1496,68 | 30,38 | | | | |
| 1507,74 | 40,02 | | | | |
| 1508,68 | 29,33 | GSPhGSPhGPDGKTGPPGPh | Collagen alpha-1 (I) chain | 542-558 | CO1A1_HUMAN |
| 1510,68 | 20,17 | | | | |
| 1513,44 | 36,79 | | | | |
| 1521,69 | 30,53 | GDSDDDEPPPLPRL | Membrane associated progesterone receptor component 1 | 54-67 | PGRC1_HUMAN |
| 1523,74 | 40,66 | GDPGPPGPhPGPhPGPhPAl | Collagen alpha-1 (XV) chain | 1093-1109 | COFA1_HUMAN |
| 1523,84 | 29,75 | VIDQSRVLNLGPIT | Uromodulin | 592-605 | UROM_HUMAN |
| 1525,48 | 37,17 | | | | |
| 1526,69 | 23,92 | | | | |
| 1549,7 | 39,49 | | | | |
| 1551,66 | 22,29 | | | | |
| 1552,5 | 37,22 | | | | |
| 1608,68 | 22,35 | | | | |
| 1608,73 | 30,93 | SGDSDDDEPPPLPRL | Membrane associated progesterone receptorcomponent 1 | 53-67 | PGRC1_HUMAN |
| 1612,76 | 23,38 | APGSKGDTGAKGEPGPVG | Collagen alpha-1 (I) chain | 1438-455 | CO1A1_HUMAN |
| 1624,55 | 37,73 | | | | |
| 1638,73 | 20,23 | AGSEADHEGTHSTKRG | Fibrinogen alpha chain | 607-622 | FIBA_HUMAN |
| 1640,58 | 23,24 | | | | |
| 1640,68 | 28,04 | | | | |
| 1654,78 | 23,13 | | | | |
| 1664,75 | 29,81 | | | | |
| 1669,69 | 21,47 | DEAGSEADHEGTHSTK | Fibrinogen alpha chain | 605-620 | FIBA_HUMAN |
| 1692,8 | 30,89 | PPhGEAGKPhGEQGVPGDLG | Collagen alpha-1 (I) chain | 651-668 | CO1A1_HUMAN |
| 1697,74 | 30,88 | NGAPGNDGAKGDAGAPGAPG | Collagen alpha-1 (I) chain | 700-719 | CO1A1_HUMAN |
| 1698,57 | 37,73 | | | | |
| 1716,77 | 28 | | | | |
| 1725,59 | 38,32 | | | | |
| 1732,77 | 28,17 | | | | |
| 1749,81 | 30,61 | GPPhGEAGKPhGEQGVPGDLG | Collagen alpha-1 (I) chain | 650-668 | CO1A1_HUMAN |
| 1750,78 | 23,83 | GPPhGPPhGKNGDDGEAGKPhG | Collagen alpha-1 (I) chain | 221-239 | CO1A1_HUMAN |
| 1764,68 | 19,91 | | | | |
| 1765,81 | 31 | GPPhGEAGKPhGEQGVPhGDLG | Collagen alpha-1 (I) chain | 650-668 | CO1A1_HUMAN |
| 1769,71 | 28,14 | | | | |
| 1793,88 | 32,37 | EEAPSLRPAPPPISGGGY | Fibronogen beta chain | 54-71 | FIBB_HUMAN |
| 1798,72 | 36,95 | | | | |
| 1809,88 | 32,3 | | | | |
| 1817,69 | 20,23 | | | | |
| 1818,83 | 30,95 | | | | |
| 1822,73 | 30,87 | | | | |
| 1823,99 | 24,4 | SVIDQSRVLNLGPITR | Uromodulin | 590-606 | UROM_HUMAN |
| 1835,71 | 19,91 | | | | |
| 1837,8 | 30,56 | | | | |
| 1840,84 | 41,18 | | | | |
| 1847,89 | 43,67 | | | | |
| 1860,83 | 21,4 | EGSPhGRDGSPhGAKGDRGET | Collagen alpha-1 (I) chain | 1021-1039 | CO1A1_HUMAN |
| 1880,9 | 43,91 | | | | |
| 1885,65 | 38,82 | | | | |
| 1892,86 | 24,33 | | | | |
| 1915,91 | 31,3 | | | | |
| 1916,77 | 20,32 | | | | |
| 1934,79 | 19,94 | | | | |
| 1942,84 | 30,96 | | | | |
| 1945 | 33,71 | | | | |
| 1962,88 | 31,81 | | | | |
| 1963,88 | 31,74 | | | | |
| 1996,79 | 20,98 | | | | |
| 2034,99 | 40,19 | | | | |
| 2039,13 | 21,78 | SGSVIDQSRVLNLGPITRK | Uromodulin | 589-607 | UROM_HUMAN |
| 2055,94 | 25,44 | | | | |
| 2058,94 | 23,15 | | | | |
| 2067,82 | 20,62 | | | | |
| 2070,92 | 25,4 | GNSGEPhGAPhGSKGDTGAKGEPGPh | Collagen alpha-1 (I) chain | 431-453 | CO1A1_HUMAN |
| 2076,95 | 21,78 | | | | |
| 2080,94 | 20,2 | DAHKSEVAHRFKDLGEEN | Serum albumin | 25-42; N- term. | ALBU_HUMAN |
| 2128,98 | 26,97 | DGKTGPhPGPAGQDGRPGPPhGPhPhG | Collagen alpha-1 (I) chain | 550-572 | CO1A1_HUMAN |
| 2132,91 | 25,83 | | | | |
| 2137,94 | 21,79 | NGEPhGGKGERGAPhGEKGEGGPhPG | Collagen alpha-1 (III) chain | 818-840 | CO3A1_HUMAN |
| 2156,97 | 22,22 | AEGSPhGRDGSPhGAKGDRGETGPA | Collagen alpha-1 (I) chain | 1020-1042 | CO1A1_HUMAN |
| 2168,97 | 32,91 | | | | |
| 2189 | 26,89 | ADGQPGAKGEPGDAGAKGDAGPPHGP | Collagen alpha-1 (I) chain | 819-843 | CO1A1_HUMAN |
| 2191,99 | 22,39 | | | | |
| 2194,97 | 20,17 | NDGPPhGRDGQPhGHKGERGYPhG | Collagen alpha-2 (I) chain | 932-952 | CO1A2_HUMAN |
| 2216,03 | 33,83 | | | | |
| 2226,99 | 26,28 | SNSGEPhGAPhGSKGDTGAKGEPhGPVG | Collagen alpha-1 (I) chain | 431-455 | CO1A1_HUMAN |
| 2235,04 | 34,17 | GRTGDAGPVGPPGPPhGPhPhGPhPGPPS | Collagen alpha-1 (I) chain | 1169-1193 | CO1A1_HUMAN |
| 2248,99 | 25,99 | | | | |
| 2249,04 | 20,53 | GKNGDDGEAGKPhGRPhGERGPhPGP | Collagen alpha-1 (I) chain | 227-249 | CO1A1_HUMAN |
| 2257,87 | 35,93 | | | | |
| 2276,02 | 27,23 | ADGQPGAKGEPhGDAGAKGDAGPPGPhA | Collagen alpha-1 (I) chain | 819-844 | CO1A1_HUMAN |
| 2277,01 | 27,23 | | | | |
| 2280,94 | 36,22 | | | | |
| 2289,04 | 33,59 | | | | |
| 2292,02 | 27,28 | ADGQPhGAKGEPhGDAGAKGDAGPPhGPA | Collagen alpha-1 (I) chain | 819-844 | CO1A1_HUMAN |
| 2308,02 | 27,34 | ADGQPhGAKGEPhGDAGAKGDAGPhPhGPA | Collagen alpha-1 (I) chain | 819-844 | CO1A1_HUMAN |
| 2319,07 | 33,82 | | | | |
| 2323,04 | 22,36 | GQNGEPhGGKGERGAPhGEKGEGGPPhG | Collagen alpha-1 (III) chain | 816-840 | CO3A1_HUMAN |
| 2339 | 34,01 | | | | |
| 2367,06 | 27,63 | | | | |
| 2377,1 | 20,8 | GKNGDDGEAGKhPGRPhGERGPPhGPQ | Collagen alpha-1 (I) chain | 227-250 | CO1A1_HUMAN |
| 2385,05 | 33,95 | | | | |
| 2421 | 34,86 | | | | |
| 2446,09 | 28,37 | ADGQPhGAKGEPhGDAGAKGDAGPhPGPAGP | Collagen alpha-1 (I) chain | 819-846 | CO1A1_HUMAN |
| 2471,16 | 34,77 | TGPIGPPhGPAGAPhGDKGESGPSGPAGPTG | Collagen alpha-1 (I) chain | 766-794 | CO1A1_HUMAN |
| 2483,12 | 27,57 | | | | |
| 2485,13 | 34,41 | | | | |
| 2525,2 | 27,74 | | | | |
| 2544,13 | 28,26 | | | | |
| 2559,18 | 19,41 | DEAGSEADHEGTHSTKRGHAKSRP | Fibrinogen alpha chain | 605-628 | FIBA_HUMAN |
| 2570,19 | 42,56 | | | | |
| 2583,15 | 23,68 | | | | |
| 2584,23 | 35,18 | | | | |
| 2587,2 | 21,1 | | | | |
| 2596,23 | 34,9 | | | | |
| 2612,21 | 34,9 | | | | |
| 2639,29 | 21,42 | | | | |
| 2644,22 | 21,15 | | | | |
| 2654,19 | 23,92 | ERGEAGIPhGVPhGAKGEDGKDGSPhGEPhGA | Collagen alpha-1 (III) chain | 448-475 | CO3A1_HUMAN |
| 2668,25 | 41,97 | | | | |
| 2748,79 | 36,38 | | | | |
| 2751,34 | 29,23 | | | | |
| 2756,27 | 35,24 | | | | |
| 2767,32 | 21,67 | KEGGKGPRGETGPAGRPhGEVGPhPGPPhGPAG | Collagen alpha-1 (I) chain | 906-935 | CO1A1_HUMAN |
| 2802,82 | 36,34 | | | | |
| 2839,35 | 24,2 | | | | |
| 2907.35 | 35,96 | | | | |
| 2912,17 | 25,56 | | | | |
| 2926,3 | 22,22 | ESGREGAPGAEGSPhGRDGSPhGAKGDRGETGP | Collagen alpha-1 (I) chain | 1011-1041 | CO1A1_HUMAN |
| 2939,15 | 33,77 | | | | |
| 2946,21 | 34,98 | | | | |
| 2973,45 | 24,37 | | | | |
| 3001,43 | 35,4 | | | | |
| 3002,24 | 23,8 | | | | |
| 3011,39 | 29,75 | LTGSPhGSPhGPhDGKTGPPGPAGQDGRPGPPhGPhPhG | Collagen alpha-1 (I) chain | 537-569 | CO1A1_HUMAN |
| 3013,29 | 22,3 | ESGREGAPhGAEGSPhGRDGSPhGAKGDRGETGPA | Collagen alpha-1 (I) chain | 1011-1040 | CO1A1_HUMAN |
| 3035,19 | 42,02 | | | | |
| 3064,32 | 20,57 | | | | |
| 3081,42 | 29,83 | | | | |
| 3091,44 | 28,4 | | | | |
| 3108,45 | 31,28 | ADGQPhGAKGEPhGDAGAKGDAGPhPGPAGPAGPPGPhIG | Collagen alpha-1 (I) chain | 819-854 | CO1A1_HUMAN |
| 3139,49 | 29,48 | | | | |
| 3149,46 | 31,25 | GADGQPGAKGEPhGDAGAKGDAGPPhGPAGPhAGPPGPIG | Collagen alpha-1 (I) chain | 818-854 | CO1A1_HUMAN |
| 3165,46 | 31,32 | | | | |
| 3178,43 | 30,3 | | | | |
| 3193,38 | 22,64 | PPhGESGREGAPGAEGSPhGRDGSPhGAKGDRGETGP | Collagen alpha-1 (I) chain | 1008-1041 | CO1A1_HUMAN |
| 3209,41 | 22,67 | PPhGESGREGAPhGAEGSPhGRDGSPhGAKGDRGETGP | Collagen alpha-1 (I) chain | 1008-1041 | CO1A1_HUMAN |
| 3256,53 | 33,03 | | | | |
| 3281,43 | 36,09 | | | | |
| 3292,54 | 39,42 | | | | |
| 3318,55 | 30,99 | | | | |
| 3333,72 | 23,83 | | | | |
| 3334,54 | 31,02 | | | | |
| 3337,45 | 22,81 | GPPhGESGREGAPhGAEGSPhGRDGSPhGAKGDRGETGPA | Collagen alpha-1 (I) chain | 1007-1042 | CO1A1_HUMAN |
| 3350,55 | 31,02 | | | | |
| 3359,58 | 31,9 | | | | |
| 3363,54 | 30,22 | | | | |
| 3385,55 | 25,49 | | | | |
| 3401,6 | 25,47 | | | | |
| 3405,48 | 25,97 | ARGNDGARGSDGQPGPPhGPhPhGTAGFPhGSPhGAKGEVGP | Collagen alpha-1 (III) chain | 319-355 | CO3A1_HUMAN |
| 3426,31 | 27,7 | | | | |
| 3575,75 | 32,36 | | | | |
| 3657,67 | 40,71 | | | | |
| 3696,76 | 26,94 | | | | |
| 3718,72 | 32,48 | | | | |
| 3734,72 | 32,5 | | | | |
| 3831,81 | 28,48 | | | | |
| 3870,81 | 33,49 | | | | |
| 3871,79 | 27,57 | | | | |
| 3943,83 | 33,63 | | | | |
| 4002,62 | 20,66 | | | | |
| 4047,92 | 25,45 | | | | |
| 4078,81 | 33,14 | | | | |
| 4217,98 | 26,05 | EEKAVADTRDQADGSRASVDSGSSEEQGGSSRALVSTLVPLG | Polymeric-immunoglobulin receptor | 607-648 | PIGR_HUMAN |
| 4251,98 | 28,76 | | | | |
| 4289,93 | 28,78 | ARGNDGARGSDGQPhGPhPhGPPGTAGFPGSPhGAKGEVGPhAGSPhGS- NGAPhG | Collagen alpha-1 (III) chain | 319-366 | CO3A1_HUMAN |
| 4368,9 | 20,21 | | | | |
| 4436,08 | 26,32 | | | | |
| 4467,96 | 29,12 | | | | |
| 4630 | 29,38 | | | | |
| 4863,16 | 26,74 | | | | |
| 8917,25 | 22,55 | | | | |

## Patentansprüche

1. Verfahren zur Diagnostik von Diabetes mellitus umfassend den Schritt der Bestimmung einer An- oder Abwesenheit oder Amplitude von mindestens drei Polypeptidmarkern in einer Urinprobe, wobei die Polypeptidmarker ausgewählt sind aus den Markern, die in Tabelle 1 durch Werte für die Molekularmassen und die Migrationszeit charakterisiert sind.

2. Verfahren nach Anspruch 1 **dadurch gekennzeichnet, dass** eine Auswertung der bestimmten An- oder Abwesenheit oder Amplitude der Marker anhand folgender Referenzwerte erfolgt:
- Differentialdiagnostik zwischen gesund und Diabetes mellitus anhand Tabelle 2

3. Verfahren nach mindestens einem der Ansprüche 1 bis 2, wobei mindestens fünf, mindestens sechs, mindestens acht, mindestens zehn, mindestens 20 oder mindestens 50 Polypeptidmarker verwendet werden, wie sie in Anspruch 1 definiert sind.

4. Verfahren nach einem der Ansprüche 1 bis 3, **dadurch gekennzeichnet, dass** die Marker so ausgewählt werden, dass der gemittelte Bonferoni korrigierte p-Wert < 10⁻⁴ ist.

5. Verfahren nach einem der Ansprüche 1 bis 4, wobei Kapillarelektrophorese, HPLC, Gasphasenionenspektrometrie und/oder Massenspektrometrie zur Bestimmung der An- oder Abwesenheit oder Amplitude der Polypeptidmarker verwendet wird.

6. Verfahren nach einem der Ansprüche 1 bis 5, wobei vor einer Messung der Molekularmasse der Polypeptidmarker eine Kapillarelektrophorese durchgeführt wird.

7. Verfahren nach einem der Ansprüche 1 bis 6, wobei Massenspektrometrie zum Nachweis der An- oder Abwesenheit oder zur Bestimmung der Amplitude des/der Polypeptidmarker verwendet wird.

8. Verwendung von mindestens drei Polypeptidmarkern ausgewählt aus den Markern gemäß Tabelle 1, die durch die Werte für die Molekularmassen und die Migrationszeit charakterisiert ist, zur Diagnostik von Diabetes mellitus.

9. Verfahren zur Diagnose von Diabetes mellitus umfassend die Schritte
a) der Auftrennung einer Probe in mindestens fünf, bevorzugt 10 Teilproben,
b) Analyse von mindestens fünf Teilproben zur Bestimmung einer An- oder Abwesenheit oder Amplitude mindestens eines Polypeptidmarkers in der Probe, wobei der Polypeptidmarker ausgewählt ist aus den Markern der Tabelle 1, die durch die Molekularmassen und Migrationszeit (CE-Zeit) charakterisiert sind.

10. Verfahren nach Anspruch 9, wobei mindestens 10 Teilproben gemessen werden.

11. Verfahren nach mindestens einem der Ansprüche 1 bis 10, **dadurch gekennzeichnet dass** die CE-Zeit bezogen ist auf eine 90 cm lange Glaskapillare mit einem inneren Durchmesser (ID) von 50 µm bei einer angelegten Spannung von 25 kV, wobei als Laufmittel 20% Acetonitril, 0,25% Ameisensäure in Wasser verwendet wird.

12. Markerkombination, umfassend mindestens 10 Marker ausgewählt aus den Markern der Tabelle 1, die durch Molekularmassen und Migrationszeit (CE-Zeit) charakterisiert sind.

13. Verfahren nach mindestens einem der Ansprüche 1 bis 7 oder 9 bis 11, wobei die Sensitivität mindestens 60% und die Spezifität mindestens 60% beträgt.
